# EUROPEAN PATENT APPLICATION

(11) **EP 4 844 007 A1**
(43) Date of publication of application: **25.11.2026**
(21) Application number: 25741594.3
(22) Date of filing: 16.01.2025
(51) Int. Cl.: C07K 19/00, C12N 15/62

(54) **TARGETED DEGRADATION SYSTEM, SCREENING METHOD THEREFOR, AND USE THEREOF**

(30) Priority: 16.01.2024 CN 202410064969
(71) Applicant: Tsinghua University, Beijing 100084 (CN)
(72) Inventor: LI, Pilong, Beijing 100084 (CN); JIA, Wen, Beijing 100084 (CN); XU, Weifan, Beijing 100084 (CN)
(74) Representative: Schulz Junghans Patentanwälte PartGmbB
(86) International application number: PCT/CN2025/072876
(87) International publication number: WO 2025/153033

(57) **Abstract**

Provided are a targeted degradation system constructed by a multispecific fusion protein containing a p62 nanobody, a method for screening the fusion protein, and a method and use for targeted degradation of a target protein by using the fusion protein.

## Description

### Technical Field

The present invention relates to a targeted degradation system constructed using a multispecific fusion protein comprising a p62 nanobody, a method for screening the fusion protein, and a method and use for targeted degradation of a target protein using the fusion protein.

### Background Art

Over the past few decades, tremendous advances have been made in the discovery of drug targets, with new targets being discovered every year. However, approximately 62% of these disease-associated target proteins are undruggable or difficult to be developed as a drug, mainly because most of them contain extensive disordered structures, making it impossible to resolve three-dimensional crystal structures suitable for drug development, or lack suitable protein-ligand interaction regions (e.g., well-defined hydrophobic pockets), making the design of specific targeted drugs against them highly challenging.

Targeted protein degradation (TPD) technology, represented by PROTAC, is considered one of the technologies most likely to achieve breakthroughs against these undruggable targets. PROTACs include various bifunctional molecules obtained by linking a target protein (POI) ligand to an E3 ligase ligand via a linker. Studies using PROTACs have found that targeted degradation is achieved by using the two targeting warheads to bring the POI and E3 ligase into physical proximity, thereby triggering polyubiquitination of the POI by the E3 ligase. This proximity may be transient, and the ligand molecule serving as a PROTAC targeting warhead need not directly inhibit POI activity or maintain prolonged, high-affinity binding to the POI. Accordingly, many small-molecule compounds whose development as target inhibitors or E3 ligase inhibitors failed can be repurposed for PROTAC development (see Liu, Z et al., An overview of PROTACs: a promising drug discovery paradigm. Mol Biomed 3, 46 (2022)). Peptide molecules (e.g., peptide substrate-binding motifs of the POI and/or E3 ligase, or antibodies against the POI and/or E3 ligase) can also specifically target the POI and E3 ligase, but are clearly not considered good choices for PROTAC targeting warheads. At least to date, all clinical-stage PROTACs have used small molecules rather than peptide ligands. One possible reason is the extremely low success rate of constructing PROTACs using peptide ligands as targeting warheads. For example, S. Matsuzawa et al. (Method for targeting protein destruction by using a ubiquitin-independent, proteasome-mediated degradation pathway. PNAS (2005), vol. 102, no. 42, 14982-14987) reported that none of the dozens of chimeric peptides generated by combining 12 different POI-binding motifs with 7 different E3-ligase- binding motifs could degrade the target protein. Another reason is that the use of peptide elements inevitably affects pharmacokinetics (e.g., poor membrane permeability and poor oral bioavailability). Accordingly, when the PROTAC concept first emerged, it was expressly stated that the ultimate goal in developing such bifunctional molecules was to identify small molecules having E3-targeting activity that could replace peptide motifs (K. M. Sakamoto et al., Protacs: Chimeric molecules that target proteins to the Skp1-Cullin-F box complex for ubiquitination and degradation. Proc Natl Acad Sci U S A. 2001 Jul 17; 98(15): 8554-8559).

In addition, bispecific antibodies have been reported as bifunctional molecules for targeted degradation. These include AbTAC technology (see CN114867752A), which has both specificity for targeting cell membrane surface E3 ligase RNF43 and specificity for targeting cell membrane surface proteins; PROTAB technology (see CN116761825A), which has both specificity for targeting cell membrane surface E3 ligase RNF43 or ZNRF3 and specificity for targeting cell membrane surface proteins; and KineTAC technology (see WO2022212593A1), which has both specificity for targeting cell membrane surface receptors (such as CXCL12) and specificity for targeting cell membrane surface or extracellular proteins. The first two technologies are still based on the ubiquitin-proteasome pathway: the two arms of the bispecific antibody bring the POI and E3 ligase into spatial proximity, after which ubiquitination and proteasomal degradation occur. The third technology exploits antibody internalization, such that the POI bound by the bispecific antibody enters an endosome upon internalization and is degraded. Thus, current bispecific-antibody-based targeted degradation technologies are mainly limited to using cell-surface E3 ligases or receptors to degrade adjacent membrane or extracellular proteins. This is likely due to the poor membrane permeability of antibody molecules, which prevents them from reaching an effective concentration within cells.

In addition to proceeding through the ubiquitin-proteasome pathway described above, targeted degradation may also proceed through the lysosomal pathway, which is further divided into the endosomelysosome pathway and the autophagy-lysosome pathway. Among them, the autophagy-lysosome pathway involves autophagosomes encapsulating intracellular targets to be degraded, which then fuse with lysosomes to form autolysosomes. Technologies based on the autophagy-lysosome pathway include AUTAC, AUTOTAC, and ATTEC. ATTEC, which is structurally similar to PROTAC, is a bifunctional molecule obtained by linking a small-molecule ligand of LC3 protein to a POI ligand via a linker. After activation, the autophagy marker LC3 protein is located on the membrane of an autophagic vesicle and is a key factor in inducing the vesicle to engulf the target protein and form an autophagosome. The autophagosome is then transported to a lysosome, thereby degrading the target protein (see Zhaoyang Lia et al., ATTEC: a potential new approach to target proteinopathies. AUTOPHAGY (2020), Vol. 16, No. 1, 185-187). Both targeting warheads of AUTAC also use small-molecule ligands, but one ligand is cysteine-S-guanine (cGMP), which can trigger K63 ubiquitination. The small molecule triggers K63 ubiquitination and thereby directs the target protein to selective autophagy (see Daiki Takahashi et al., AUTACs: Cargo-Specific Degraders Using Selective Autophagy. Molecular Cell (2019), Vol. 76, No.5, P797-810.E10). AUTOTAC uses, as one targeting warhead, a peptide motif that specifically recognizes p62 protein. The peptide motif must not only specifically recognize and bind p62 protein, but also activate the normally inactive p62 protein and induce a conformational change. Conformationally altered p62 protein can self-condense and specifically bind LC3 protein, enter an LC3-induced autophagosome, and ultimately cause degradation of the target protein bound by AUTOTAC.

Compared to the ubiquitin-proteasome pathway, one of the advantages of the autophagy-lysosome pathway is that the molecular size of the targets it degrades is not limited by the proteasome pore size. Moreover, this pathway can degrade not only soluble proteins or membrane proteins, but also many targets including extracellular proteins, protein aggregates, organelles and non-proteins (such as lipid droplets), and has a wider range of applications than the ubiquitin-proteasome pathway. However, whether in view of the lack of an accumulated repertoire of inhibitory ligands for autophagy markers comparable to that available for ubiquitin ligases, or the great difficulty of developing specific ligands for difficult-to-drug targets (especially proteins dominated by disordered structures) (Joshi, P., Vendruscolo, M. (2015). Druggability of Intrinsically Disordered Proteins. In: Felli, I., Pierattelli, R. (eds) Intrinsically Disordered Proteins Studied by NMR Spectroscopy. Advances in Experimental Medicine and Biology, vol 870. Springer, Cham.), it is evident that constructing high-throughput libraries of such bifunctional molecules is difficult, severely constraining related drug development. Furthermore, LC3 and p62 proteins play roles in their respective pathways that differ from those of E3 ligases. As described above, in order to achieve targeted degradation, a PROTAC merely need to bring a target protein (serving as the ubiquitination substrate) into physical proximity with an E3 ligase to facilitate the enzymatic ubiquitination reaction. This, however, is clearly insufficient for the above-mentioned bifunctional molecules targeting autophagy-pathway proteins (particularly AUTOTAC molecules targeting p62 protein) to perform their targeted degradation function.

"Liquid-Liquid Phase Separation" (LLPS, also referred to herein as "phase separation") describes the process by which multivalent biomacromolecules aggregate through intermolecular or intramolecular interactions, thereby being separated from the surrounding bulk solution phase and forming an independent liquid phase enriched in the biomacromolecules. It may also be referred to as a "phase transition." After LLPS occurs, numerous small droplets enriched in the biomacromolecules can be observed within cells, with diameters of several micrometers or even larger. Such readily recognizable small droplets are referred to as "phase-transition droplets." It has been reported that activated p62 and polyubiquitinated proteins are key to the condensation of p62 bodies, which serve as nucleation sites in selective autophagy (see Agudo-Canalejo J et al., Wetting regulates autophagy of phase-separated compartments and the cytosol. Nature. 2021 Mar;591(7848):142-146; Turco E et al. FIP200 Claw Domain Binding to p62 Promotes Autophagosome Formation at Ubiquitin Condensates. Mol Cell. 2019 Apr 18;74(2):330-346.e11). However, it has not been reported that a protein that is itself prone to LLPS (e.g., an intrinsically disordered protein) can directly trigger autophagyto achieve targeted degradation of the target protein, without relying on p62 activation and/or polyubiquitination.

### Summary of the Invention

Through extensive research, the inventors found that a multispecific fusion protein having specificity for both the autophagy receptor p62 protein and an intrinsically disordered protein or a protein having an intrinsically disordered region can exploit the aggregation propensity of the intrinsically disordered protein to promote the condensation required for p62 activation. This promotes p62 activation, thereby allowing the degradation of the specifically targeted intrinsically disordered protein or protein having an intrinsically disordered region via the autophagy pathway, and the regulation of biological activities or signaling pathways in which that protein participates.

Accordingly, one object of the present invention is to provide a multispecific fusion protein comprising at least one first affinity peptide that specifically binds to an autophagy receptor p62 protein and at least one second affinity peptide that specifically binds to a target, wherein the first affinity peptide and the second affinity peptide are covalently linked through a linking sequence/linker. In some embodiments, the first affinity peptide and the second affinity peptide are directly linked by a covalent bond.

In some embodiments, the multispecific fusion protein disclosed herein is capable of forming, in the presence of p62 protein and the target, a ternary complex comprising the fusion protein, p62 protein, and the target. In some embodiments, the multispecific fusion protein disclosed herein increases liquid-liquid phase separation of p62 protein relative to a condition in which the fusion protein is absent. In some embodiments, the increase in liquid-liquid phase separation is selected from an increase in the number of liquid-liquid phase-separation droplets containing p62 protein, an increase in the area of liquid-liquid phase-separation droplets containing p62 protein, an increase in the degree of oligomerization of p62 protein in liquid-liquid phase-separation droplets containing p62 protein, a decrease in the fluidity of p62 protein in liquid-liquid phase-separation droplets containing p62 protein, or any combination thereof. In some embodiments, the target is an intrinsically disordered protein or a protein aggregate, membrane complex, and/or organelle containing an intrinsically disordered protein as a monomer or component. In some embodiments, the target is selected from a cytoplasmic protein, a nuclear protein, a membrane protein, or an organelle. In some embodiments, the target is selected from TDP43, hnRNPK, G3BP1, HTT-Q103, TSPAN4, PD-L1, APP, or STAT3.

In some embodiments, the first affinity peptide and the second affinity peptide of the multispecific fusion protein disclosed herein are each independently selected from a nanobody or a synthetic binding protein. In some embodiments, the nanobody is selected from a single-domain antibody, a single-chain antibody (scFv), a minibody, a half-antibody, or an antigen-binding fragment of an antibody. In some embodiments, the synthetic binding protein is selected from a monobody, an affibody, an anticalin, or a DARPin. In a preferred embodiment, the single-domain antibody is selected from a V_{H}H domain antibody, a heavy-chain variable domain (VH) antibody, a V_{NAR} domain antibody, and a V_{L} domain antibody.

In some embodiments, the first affinity peptide comprises CDR1, CDR2, and CDR3 contained in any one of SEQ ID NO 9, 11, 13, 67, or 72. In a preferred embodiment, the first affinity peptide comprises CDR1, CDR2 and CDR3 having the amino acid sequences set forth in SEQ ID NOs 51, 52 and 53, respectively. In some other preferred embodiments, the first affinity peptide comprises CDR1, CDR2 and CDR3 having the amino acid sequences set forth in SEQ ID NOs 54, 55 and 56, respectively. In some other preferred embodiments, the first affinity peptide comprises CDR1, CDR2 and CDR3 having the amino acid sequences set forth in SEQ ID NOs 57, 58 and 59, respectively. In some other preferred embodiments, the first affinity peptide comprises CDR1, CDR2 and CDR3 having the amino acid sequences set forth in SEQ ID NOs 68, 69 and 70, respectively. In some other preferred embodiments, the first affinity peptide comprises CDR1, CDR2 and CDR3 having the amino acid sequences set forth in SEQ ID NOs 73, 74 and 75, respectively. In some embodiments, the first affinity peptide comprises the amino acid sequence set forth in any one of SEQ ID NOs 9, 11, 13, 67, and 72. In some embodiments, the first affinity peptide comprises an amino acid sequence having at least 90%, 95%, 98%, or 99% identity to an amino acid sequence set forth in any one of SEQ ID NOs 9, 11, 13, 67, and 72. In some embodiments, the first affinity peptide comprises an amino acid sequence having one or several (e.g., 1, 2, 3, 4, 5, 6, 7, 8, 9, or 10) deletions, substitutions, or additions relative to an amino acid sequence set forth in any one of SEQ ID NOs 9, 11, 13, 67, and 72.

In some embodiments, the second affinity peptide comprises CDR1, CDR2, and CDR3 contained in SEQ ID NO 19 or 77. In a preferred embodiment, the second affinity peptide comprises the amino acid sequence set forth in SEQ ID NO 19 or 77. In some embodiments, the second affinity peptide comprises an amino acid sequence having at least 90%, 95%, 98%, or 99% identity to the amino acid sequence set forth in SEQ ID NO 19 or 77. In some embodiments, the second affinity peptide comprises an amino acid sequence having one or several (e.g., 1, 2, 3, 4, 5, 6, 7, 8, 9, or 10) deletions, substitutions, or additions relative to the amino acid sequence set forth in SEQ ID NO 19 or 77.

In some embodiments, the fusion protein disclosed herein further comprises an oligomerization element. In some embodiments, the oligomerization element is directly covalently linked to the first affinity peptide and/or the second affinity peptide. In some embodiments, the oligomerization element is covalently linked to the first affinity peptide and/or the second affinity peptide through a linking sequence/linker. In some embodiments, the oligomerization element is derived from an oligomerization domain of a protein capable of oligomerization. In some embodiments, the oligomerization element is an artificially constructed coiled-coil domain. In some embodiments, the oligomerization element enables the fusion protein to form oligomers ranging from dimers to 60-mers. In some embodiments, the oligomerization element is a dimeric or higher-order oligomerization domain. In a preferred embodiment, the oligomerization element is a tetrameric or higher-order oligomerization domain. In a more preferred embodiment, the oligomerization element is an octameric or higher-order oligomerization domain. In the most preferred embodiment, the oligomerization element is a 12-mer or higher-order oligomerization domain. In some embodiments, each fusion protein comprises one or more oligomerization elements. In a preferred embodiment, each fusion protein comprises one oligomerization element. In some embodiments, the oligomerization element comprises the amino acid sequence set forth in any one of SEQ ID NOs 60 to 62. In some embodiments, the oligomerization element comprises an amino acid sequence having at least 90%, at least 91%, at least 92%, at least 93%, at least 94%, at least 95%, at least 96%, at least 97%, at least 98%, or at least 99% identity to an amino acid sequence set forth in any one of SEQ ID NOs 60 to 62. In some embodiments, the oligomerization element comprises an amino acid sequence having one or several deletions, substitutions, or additions relative to an amino acid sequence set forth in any one of SEQ ID NOs 60 to 62, for example, 1, 2, 3, 4, 5, 6, 7, 8, 9, or 10 deletions, substitutions, or additions.

In some embodiments, the fusion protein disclosed herein further comprises a phase-separation element. In some embodiments, the phase-separation element is directly covalently linked to the first affinity peptide and/or the second affinity peptide. In some embodiments, the phase-separation element is covalently linked to the first affinity peptide and/or the second affinity peptide through a linking sequence/linker. In some embodiments, each fusion protein comprises one or more phase-separation elements, for example, 1, 2, 3, 4, 5, 6, 7, 8, 9, or 10 phase-separation elements. In some embodiments, the phase-separation units are tandemly linked to one another. In some embodiments, the phase-separation element is selected from an intrinsically disordered region of a protein having phase-separation capability or tandemly repeated interaction domains having phase-separation capability. In some embodiments, the phase-separation element comprises the amino acid sequence set forth in any one of SEQ ID NOs 63 to 66. In some embodiments, the phase-separation element comprises an amino acid sequence having at least 90%, at least 91%, at least 92%, at least 93%, at least 94%, at least 95%, at least 96%, at least 97%, at least 98%, or at least 99% identity to an amino acid sequence set forth in any one of SEQ ID NOs 63 to 66. In some embodiments, the phase-separation element comprises an amino acid sequence having one or several deletions, substitutions, or additions relative to an amino acid sequence set forth in any one of SEQ ID NOs 63 to 66, for example, 1, 2, 3, 4, 5, 6, 7, 8, 9, or 10 deletions, substitutions, or additions.

In some embodiments, the linking sequence or linker in the multispecific fusion protein disclosed herein is a rigid linker or a flexible linker. In a preferred embodiment, the linking sequence or linker is a flexible linker. In a more preferred embodiment, the linking sequence or linker is a linker comprising the sequence set forth in SEQ ID NO 50.

In some embodiments, the multispecific fusion protein disclosed herein comprises one first affinity peptide and one second affinity peptide. In some embodiments, the multispecific fusion protein disclosed herein comprises one first affinity peptide and two or more identical or different second affinity peptides. In some embodiments, the multispecific fusion protein disclosed herein comprises two or more identical or different first affinity peptides and one second affinity peptide. In some embodiments, the multispecific fusion protein disclosed herein comprises two or more identical or different first affinity peptides and two or more identical or different second affinity peptides.

Another object of the present invention is to provide a nucleic acid comprising a nucleotide sequence encoding a multispecific fusion protein disclosed herein. In some embodiments, the encoding nucleotide sequence is operably linked to a promoter.

Another object of the present invention is to provide a recombinant vector comprising a nucleic acid disclosed herein. In some embodiments, the recombinant vector is a recombinant viral vector.

Another object of the present invention is to provide an engineered cell that expresses a fusion protein disclosed herein or comprises a nucleic acid or recombinant vector disclosed herein.

Another object of the present invention is to provide a method for producing a multispecific fusion protein disclosed herein, the method comprising expressing a nucleic acid or recombinant vector disclosed herein in a suitable host cell, or culturing an engineered cell disclosed herein, and purifying the fusion protein.

Another object of the present invention is to provide a composition comprising a multispecific fusion protein, nucleic acid, recombinant vector, or engineered cell disclosed herein.

Another object of the present invention is to provide a method for degrading a target in a cell, the method comprising causing the cell to express a multispecific fusion protein disclosed herein or to comprise a nucleic acid or recombinant vector disclosed herein, such that the amount of the target is reduced relative to a condition in which the fusion protein, nucleic acid, or recombinant vector is absent. In a preferred embodiment, the method disclosed herein reduces the amount of the target by at least about 30% to about 90%.

Another object of the present invention is to provide a method for preventing and/or treating a disease, the method comprising administering to a subject a multispecific fusion protein, nucleic acid, recombinant vector, engineered cell, or composition disclosed herein.

Another object of the present invention is to provide use of a multispecific fusion protein, nucleic acid, recombinant vector, engineered cell, or composition disclosed herein in the preparation of a medicament for preventing and/or treating a disease.

In some embodiments, the disease to be prevented and/or treated is selected from those related to protein misfolding, misaggregation, or misproduction. In a preferred embodiment, the disease to be prevented and/or treated is selected from neurodegenerative diseases, cardiovascular diseases, neuromuscular diseases, tumors, metabolic diseases, or autoimmune diseases. In some embodiments, the neurodegenerative disease is selected from Alzheimer's disease, Huntington's disease, Parkinson's disease, amyotrophic lateral sclerosis, hereditary ataxia, Vici syndrome, or BPAN syndrome. In some embodiments, the cardiovascular disease is selected from coronary artery disease, atherosclerosis, or pulmonary hypertension. In some embodiments, the neuromuscular disease is selected from hereditary cardiomyopathy, distal myopathy, muscular dystrophy, congenital myopathy, spinal muscular atrophies (SMAs), motor neuron disease, Duchenne muscular dystrophy, Becker muscular dystrophy, limb-girdle muscular dystrophy, myotonic dystrophy, myotubular myopathy, centronuclear myopathy, nemaline myopathy, selenoprotein N-related myopathy, Pompe disease, glycogen storage disease III, or amyotrophic lateral sclerosis. In some embodiments, the tumor is selected from lung cancer, colorectal cancer, head and neck cancer (e.g., head and neck squamous cell carcinoma), glioma (e.g., neuroglioma), neuroblastoma, melanoma, breast cancer, bladder cancer, kidney cancer, ovarian cancer, pancreatic cancer, cervical cancer, esophageal cancer, sarcoma, esophageal cancer (e.g., esophageal squamous cell carcinoma), thyroid cancer (e.g., papillary thyroid carcinoma), prostate cancer, B-cell lymphoma, chronic lymphocytic leukemia (CLL), chronic myeloid leukemia, diffuse large B-cell lymphoma (DLBCL), follicular lymphoma, myelodysplastic syndrome (MDS), non-Hodgkin lymphoma (NHL), acute lymphoblastic leukemia (ALL), acute monocytic leukemia, multiple myeloma, acute myeloid leukemia (AML), mixed-lineage leukemia, NUT midline carcinoma, Burkitt lymphoma, mycosis fungoides (MF), or a metastatic form thereof. In some embodiments, the metabolic disease is selected from hyperlipidemia, atherosclerosis, non-alcoholic fatty liver disease, or diabetes. In some embodiments, the autoimmune disease is selected from systemic lupus erythematosus, atopic dermatitis, myasthenia gravis, or type I diabetes.

Another object of the present invention is to provide a method for screening multispecific fusion proteins, the method comprising the following steps: (a) obtaining one or more first affinity peptides capable of specifically binding to an autophagy receptor p62 protein and one or more second affinity peptides capable of specifically binding to a target, and linking at least one of the first affinity peptides to at least one of the second affinity peptides to obtain a candidate fusion protein library; (b) selecting, from the library, a candidate fusion protein that significantly increases colocalization of p62 protein with the target relative to a control in which the candidate fusion protein is absent; and (c) selecting, from the candidate fusion proteins selected in step (b), a fusion protein that increases liquid-liquid phase separation of p62 protein relative to a control in which the candidate fusion protein is absent.

In some embodiments, in step (c) of the screening method disclosed herein, the increase in liquid-liquid phase separation is selected from an increase in the number of liquid-liquid phase-separation droplets containing p62 protein, an increase in the area of liquid-liquid phase-separation droplets containing p62 protein, an increase in the degree of oligomerization of p62 protein in liquid-liquid phase-separation droplets containing p62 protein, a decrease in the fluidity of p62 protein in liquid-liquid phase-separation droplets containing p62 protein, or any combination thereof.

In some embodiments, in the screening method disclosed herein, the target is an intrinsically disordered protein or a protein aggregate, membrane complex, and/or organelle containing an intrinsically disordered protein as a monomer or component. In some embodiments, in the screening method disclosed herein, the target is selected from a cytoplasmic protein, a nuclear protein, or an organelle. In some embodiments, the target is selected from TDP43, hnRNPK, G3BP1, HTT-Q103, TSPAN4, PD-L1, APP, or STAT3.

In some embodiments, the screening method disclosed herein further comprises step (d) of determining the level of the target and selecting a fusion protein for which the amount of the target is reduced relative to a control in which the candidate fusion protein is absent. In a preferred embodiment, the fusion protein selected in step (d) reduces the amount of the target by at least about 30% to about 90%.

In some embodiments, the linking in step (a) comprises directly covalently linking the first affinity peptide to the second affinity peptide. In some embodiments, the linking in step (a) comprises covalently linking the first affinity peptide to the second affinity peptide through a linking sequence or linker. In some embodiments, the linking in step (a) further comprises linking the first affinity peptide and/or the second affinity peptide to an oligomerization element or phase-separation element. In some embodiments, in step (a), an oligomerization element or phase-separation element is covalently linked to an affinity peptide or to another such element through a linking sequence or linker.

In some embodiments, the first affinity peptide and the second affinity peptide in the screening method disclosed herein are each independently selected from a nanobody or a synthetic binding protein. In some embodiments, the nanobody is selected from a single-domain antibody, a single-chain antibody (scFv), a minibody, a half-antibody, or an antigen-binding fragment of an antibody. In a preferred embodiment, the single-domain antibody is selected from a V_{H}H domain antibody, a heavy-chain variable domain (VH) antibody, a V_{NAR} domain antibody, or a V_{L} domain antibody. In some embodiments, the synthetic binding protein is selected from a monobody, an affibody, an anticalin, or a DARPin.

In some embodiments, the first affinity peptide comprises CDR1, CDR2, and CDR3 contained in any one of SEQ ID NO 9, 11, 13, 67, or 72. In a preferred embodiment, CDR1, CDR2, and CDR3 comprised in the first affinity peptide respectively have the amino acid sequences set forth in SEQ ID NOs 51, 52, and 53. In some other preferred embodiments, CDR1, CDR2, and CDR3 comprised in the first affinity peptide respectively have the amino acid sequences set forth in SEQ ID NOs 54, 55, and 56. In some other preferred embodiments, CDR1, CDR2, and CDR3 comprised in the first affinity peptide respectively have the amino acid sequences set forth in SEQ ID NOs 57, 58, and 59. In some other preferred embodiments, CDR1, CDR2, and CDR3 comprised in the first affinity peptide respectively have the amino acid sequences set forth in SEQ ID NOs 68, 69, and 70. In some other preferred embodiments, CDR1, CDR2, and CDR3 comprised in the first affinity peptide respectively have the amino acid sequences set forth in SEQ ID NOs 73, 74, and 75.

In some embodiments, the second affinity peptide comprises CDR1, CDR2, and CDR3 contained in SEQ ID NO 19 or 77. In a preferred embodiment, the second affinity peptide comprises the amino acid sequence set forth in SEQ ID NO 19 or 77.

In some embodiments, the linking sequence or linker is a rigid linker or a flexible linker. In a preferred embodiment, the linking sequence or linker is a flexible linker. In a more preferred embodiment, the linking sequence or linker is a linker comprising the sequence set forth in SEQ ID NO 50.

In some embodiments, a candidate fusion protein in the library obtained in step (a) may comprise one first affinity peptide and one second affinity peptide; one first affinity peptide and two or more identical or different second affinity peptides; two or more identical or different first affinity peptides and one second affinity peptide; or two or more identical or different first affinity peptides and two or more identical or different second affinity peptides.

Another object of the present invention is to provide a fusion protein obtained by the screening method disclosed herein.

Another object of the present invention is to provide an affinity peptide disclosed herein, comprising CDR1, CDR2, and CDR3 contained in any one of SEQ ID NOs 9, 11, 13, 67, and 72. In some embodiments, CDR1, CDR2, and CDR3 of the first affinity peptide respectively have the amino acid sequences set forth in SEQ ID NOs 51, 52, and 53. In some other preferred embodiments, CDR1, CDR2, and CDR3 comprised in the first affinity peptide respectively have the amino acid sequences set forth in SEQ ID NOs 54, 55, and 56. In some other preferred embodiments, CDR1, CDR2, and CDR3 comprised in the first affinity peptide respectively have the amino acid sequences set forth in SEQ ID NOs 57, 58, and 59. In some other preferred embodiments, CDR1, CDR2, and CDR3 comprised in the first affinity peptide respectively have the amino acid sequences set forth in SEQ ID NOs 68, 69, and 70. In some other preferred embodiments, CDR1, CDR2, and CDR3 comprised in the first affinity peptide respectively have the amino acid sequences set forth in SEQ ID NOs 73, 74, and 75.

Another object of the present invention is to provide a nucleic acid molecule encoding an affinity peptide disclosed herein, the nucleic acid molecule comprising the nucleotide sequence set forth in any one of SEQ ID NOs 10, 12, 14, 71, and 76.

Another object of the present invention is to provide a recombinant vector comprising a nucleic acid molecule disclosed herein, an engineered cell expressing a nanobody disclosed herein or comprising a nucleic acid molecule or recombinant vector disclosed herein, and a composition comprising a nanobody, nucleic acid molecule, recombinant vector, or engineered cell disclosed herein.

### Brief Description of the Drawings

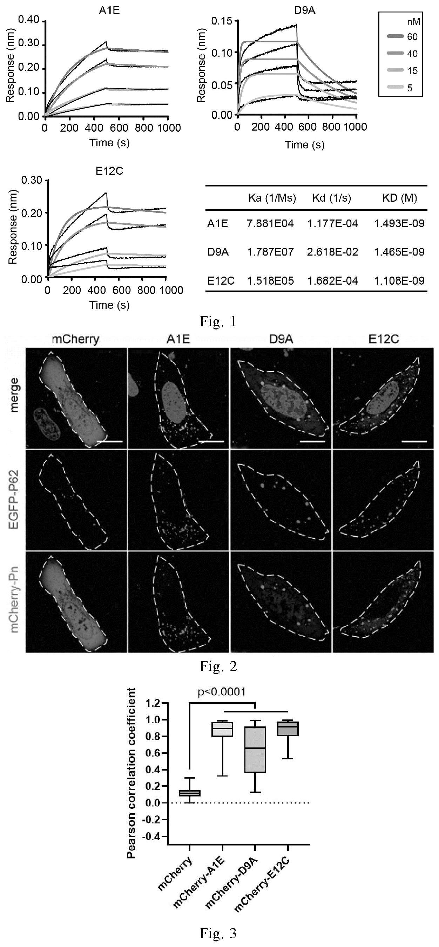

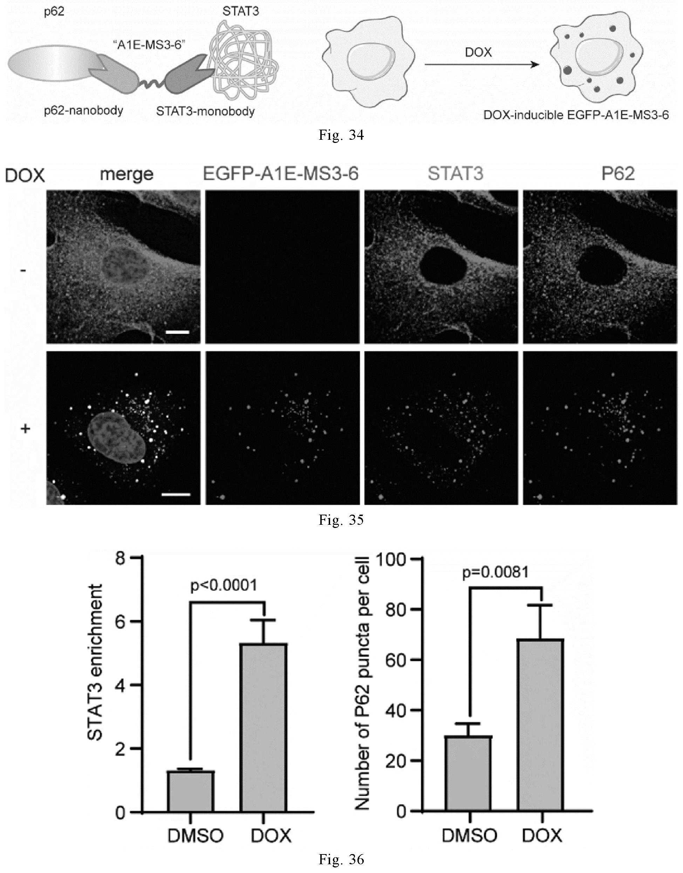

Fig. 1 illustrates the results of bio-layer interferometry (BLI) detection of interactions between p62 nanobodies and p62 protein. The horizontal axis represents reaction time, and the vertical axis represents the displacement of the interference spectrum. The black curves are the actual detection results, and the red curves are the fitted results. The table on the right shows the association constant (Ka), dissociation constant (Kd), and equilibrium dissociation constant (KD) for binding of the three nanobodies to p62.
Fig. 2 illustrates laser confocal microscopy live-cell imaging snapshots of U-2OS cells transiently co-transfected with a construct encoding a p62 nanobody (Pn) fused to the mCherry fluorescent tag and a construct encoding p62 fused to the EGFP fluorescent tag, and of control U-2OS cells transiently co-transfected with a construct encoding the mCherry fluorescent tag alone and the EGFP-p62 construct. mCherry-Pn and EGFP-p62 respectively show fluorescence images of exogenous Pn and p62. Merge is the result obtained by overlaying the two images with the nucleus (blue) in situ. A1E, D9A, and E12C respectively represent the three p62 nanobodies. The scale bar in each inset is 10 µm.
Fig. 3 illustrates colocalization statistics obtained by Pearson colocalization analysis of the images in Fig. 2. The horizontal axis represents treatment with the mCherry fluorescent tag as a control or with one of the three Pn constructs, and the vertical axis represents the Pearson colocalization coefficient calculated for the corresponding treatment condition. The statistics shown are the mean ± standard error of the mean from three independent experiments, and p-values were calculated by a two-tailed t-test.

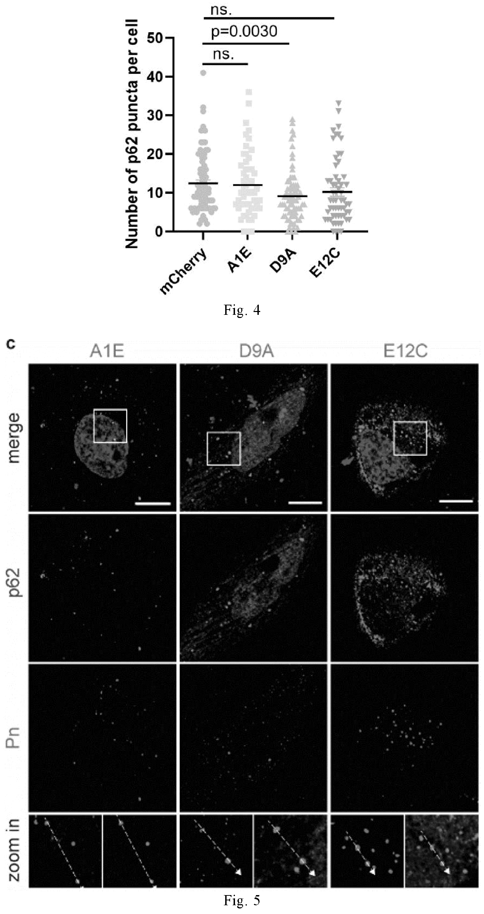

Fig. 4 illustrates the number of p62 droplets measured in Example 2.
Fig. 5 illustrates laser confocal microscopy live-cell imaging snapshots of U-2OS cells transiently transfected with mCherry-Pn. mCherry-Pn and p62 respectively show fluorescence images of exogenous Pn and endogenous p62. Merge is the result obtained by overlaying the two images with the nucleus (blue) in situ. A1E, D9A, and E12C respectively represent the three p62 nanobodies. The scale bar in each inset is 10 µm. Zoom-in is an enlarged view of the white-boxed region and shows the live-cell imaging results of colocalization analysis of the fluorescence signals representing mCherry-Pn and p62 in the puncta marked by white arrows.

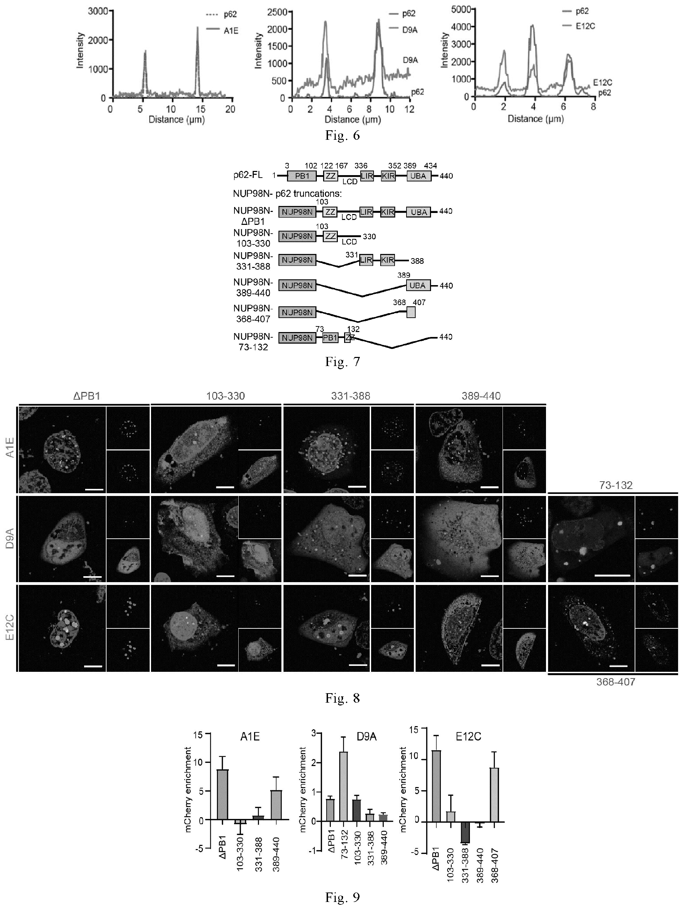

Fig. 6 illustrates statistics for the grayscale intensities of the fluorescence signals measured along the white arrows in Fig. 5. The horizontal axis represents distance along the direction indicated by each arrow, and the vertical axis represents the relative fluorescence intensity measured at the corresponding position.
Fig. 7 illustrates the structure of p62 protein and the structures of the constructs used in the CoPIC method.
Fig. 8 illustrates a laser confocal microscopy live-cell imaging snapshots of U-2OS cells transiently co-transfected with an mCherry-Pn plasmid and constructs encoding p62 truncation variants fused to EGFP-NUP98N. A1E, D9A, and E12C respectively represent the three Pns and are shown in red. ΔPB1, 103-330, 331-388, 389-440, 368-407, and 73-132 respectively represent six NUP98N-fused p62 truncation variants and are shown in green. The images containing scale bars are the results obtained by overlaying the two images with the nucleus (blue) in situ. The scale bar in each inset is 10 µm.
Fig. 9 illustrates statistics for enrichment of the mCherry-Pn fluorescence signal in NUP98N puncta under the respective treatment conditions in Fig. 8. The horizontal axis represents the different NUP98N-fused p62 truncation variants, and the vertical axis represents enrichment of the mCherry-Pn fluorescence signal in NUP98N droplets. The statistics shown are the mean ± standard error of the mean from three independent experiments.

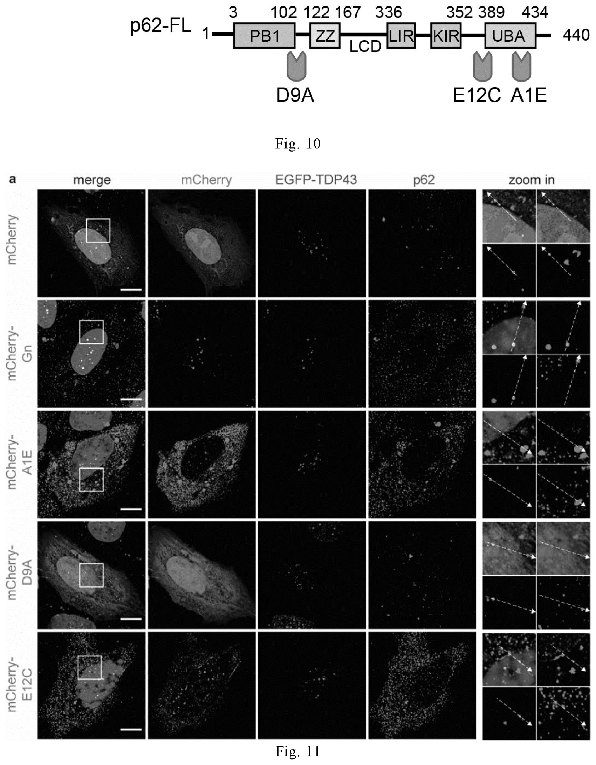

Fig. 10 illustrates a schematic diagram of the three nanobodies binding to different sites on p62 protein;
Fig. 11 illustrates a snapshot of live-cell imaging using laser confocal microscopy of U-2OS cells stably expressing EGFP-TDP43 transfected with a specified construct. Here, mCherry, EGFP-TDP43, and p62 are shown as fluorescence excitation images of the exogenous specified construct, TDP43, and endogenous p62, respectively. Merge is the result of overlaying the three images with the cell nucleus (blue) in situ. The scale bar in each inset is 10 µm. Zoom-in is a magnified view of the white-framed area in Fig. 11, showing the live-cell imaging results of colocalization analysis of fluorescence signals representing the mCherry fusion construct, EGFP-TDP43, and p62 in the puncta marked by the white arrows.

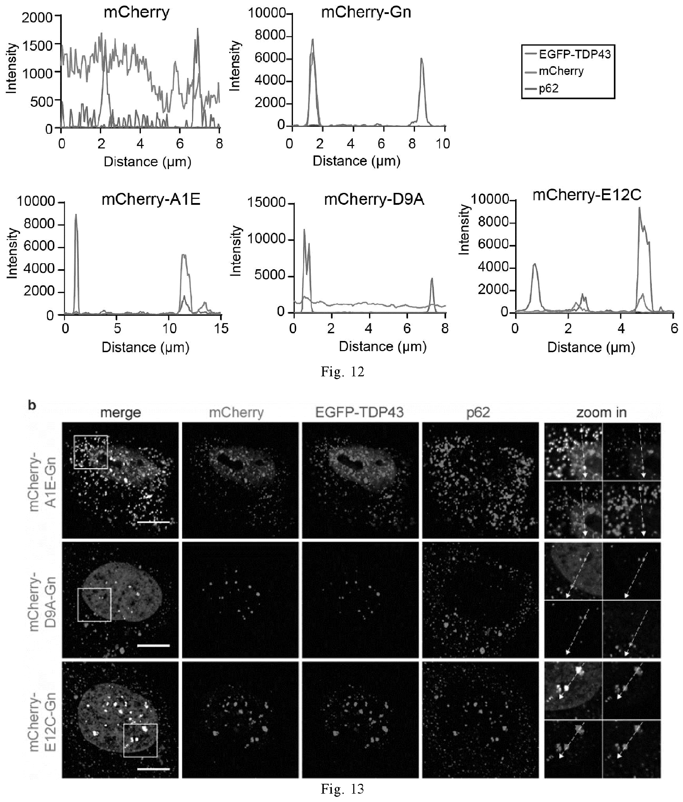

Fig. 12 illustrates the statistical results of the fluorescence signal grayscale values measured at the white arrow marks in Fig. 11; the horizontal axis in the figure represents the distance along the direction indicated by the arrow mark, and the vertical axis represents the relative fluorescence intensity measured at the corresponding position.
Fig. 13 illustrates a laser confocal microscopy live-cell imaging snapshot of U-2OS cells stably expressing EGFP-TDP43 transfected with a mCherry-Pn fused GFP nanobody (Gn) construct. The images show the fluorescence excitation of the exogenous mCherry-Pn-Gn construct, TDP43, and endogenous p62, respectively. Merge represents the result of overlaying these three images with the cell nucleus (blue) in situ. mCherry-A1E-Gn, mCherry-D9A-Gn, and mCherry-E12C-Gn represent three mCherry-Pn-Gn constructs. The scale bar in each inset is 10 µm. Zoom-in is a magnified image of the white-framed area in Fig. 13, showing the live-cell imaging results of colocalization analysis of fluorescence signals representing mCherry-Pn-Gn, EGFP-TDP43, and p62 in the puncta marked by white arrows.

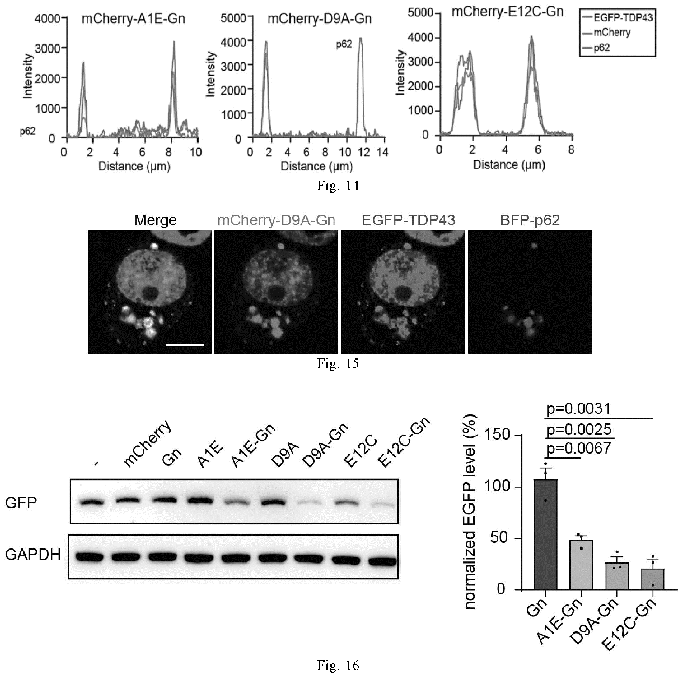

Fig. 14 illustrates the statistical results of the fluorescence signal grayscale values measured at the white arrow marks in Fig. 13; the horizontal axis in the figure represents the distance along the direction indicated by the arrow mark, and the vertical axis represents the relative fluorescence intensity measured at the corresponding position.
Fig. 15 illustrates a laser confocal microscopy live-cell imaging snapshot of HEK-293T cells co-transfected with mCherry-D9A-Gn, EGFP-TDP43, and BFP-p62 constructs. mCherry-D9A-Gn, EGFP-TDP43, and BFP-p62 respectively show fluorescence images of exogenous mCherry-D9A-Gn, EGFP-TDP43, and BFP-p62. Merge is the result obtained by overlaying the three images in situ. The scale bar is 10 µm.
Fig. 16 illustrates the results of measuring EGFP-TDP43 levels by Western blotting in HEK-293T cells stably expressing EGFP-TDP43 at 48 hours after transfection with the specified constructs. A rabbit anti-GFP polyclonal antibody was used for detection, and glyceraldehyde-3-phosphate dehydrogenase (GAPDH) was used as an internal control. Fig. 16 also shows statistics for the grayscale intensities of the Western blot bands. The horizontal axis represents the specified construct treatment conditions, and the vertical axis represents the ratio of the band grayscale intensity for the specified construct treatment to that for the mCherry treatment. All grayscale intensities were normalized to the internal-control band. The statistics shown are the mean ± standard error of the mean from three independent experiments, and the p-value was calculated by a two-tailed t-test.

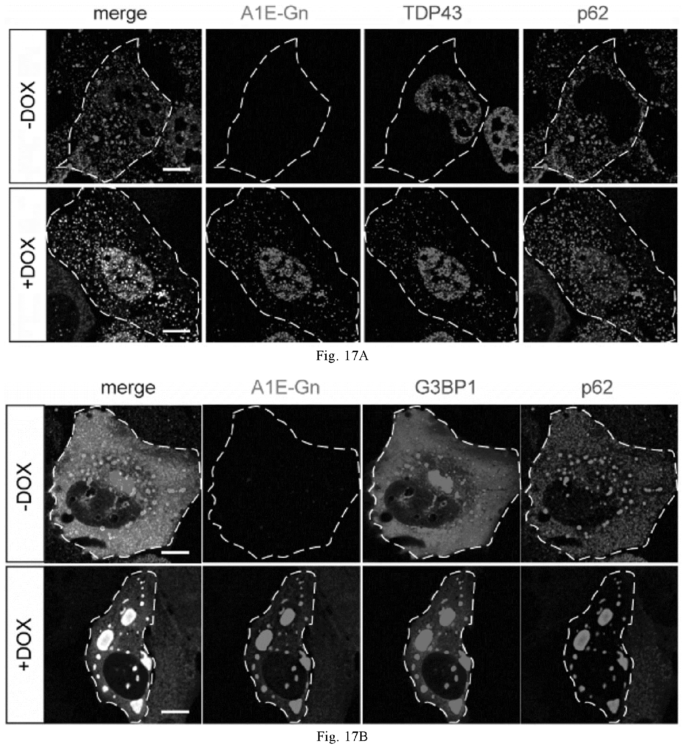

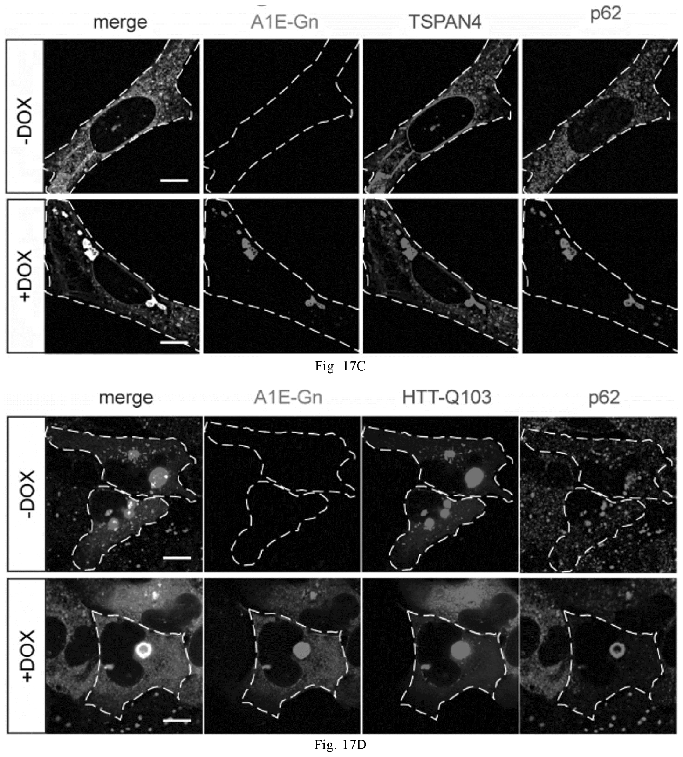

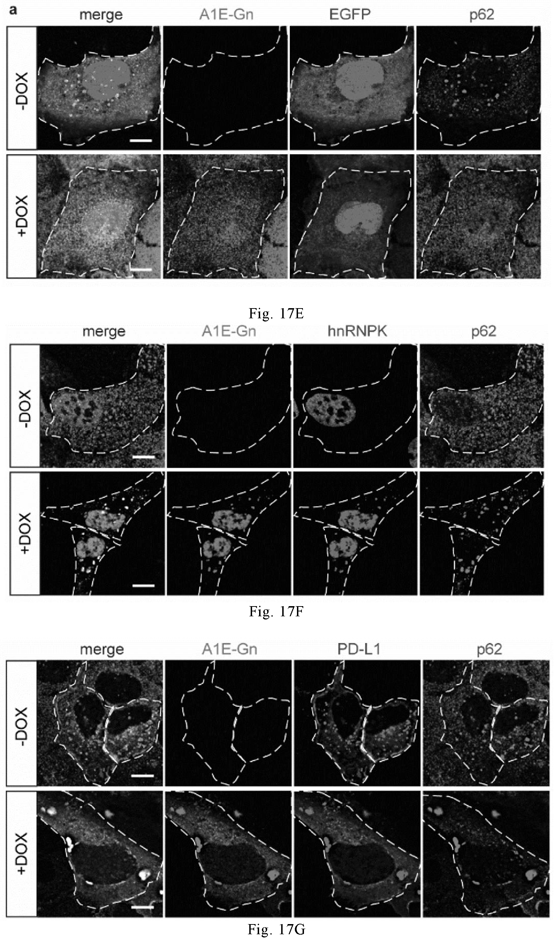

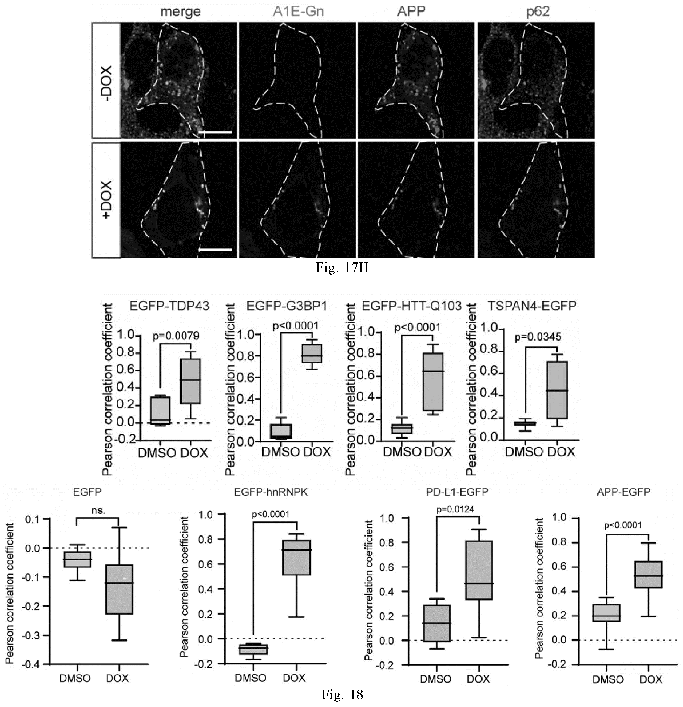

Figs. 17A to 17H illustrate laser confocal microscopy live-cell imaging snapshots of U-2OS cells that stably and inducibly express mCherry-A1E-Gn and were transfected with different specified EGFP fusion constructs or the EGFP control. A1E-Gn and p62 respectively show fluorescence images of exogenous mCherry-A1E-Gn and endogenous p62. Merge is the result obtained by overlaying these two images with the specified EGFP fusion image in situ. -DOX indicates that the cells were treated with dimethyl sulfoxide (DMSO) for 24 hours after transfection, and +DOX indicates that the cells were treated with 1 µg/mL doxycycline (DOX) for 24 hours after transfection. The scale bar in each inset is 10 µm, and white dashed lines indicate cell outlines. The specified EGFP fusions in Figs. 17A to 17H are EGFP-TDP43, EGFP-G3BP1, TSPAN4-EGFP, EGFP-HTT-Q103, the EGFP control, EGFP-hnRNPK, EGFP-PD-L1, and EGFP-APP, respectively.
Fig. 18 illustrates the colocalization statistics obtained from Pearson colocalization analysis of the images in Figs. 17A to 17H; the horizontal axis represents the specified treatment conditions, and the vertical axis represents the Pearson colocalization coefficients calculated for the corresponding treatment conditions; the statistical results shown are the mean ± standard error of the mean of three independent experiments, and the p-values were calculated by a two-tailed t-test.

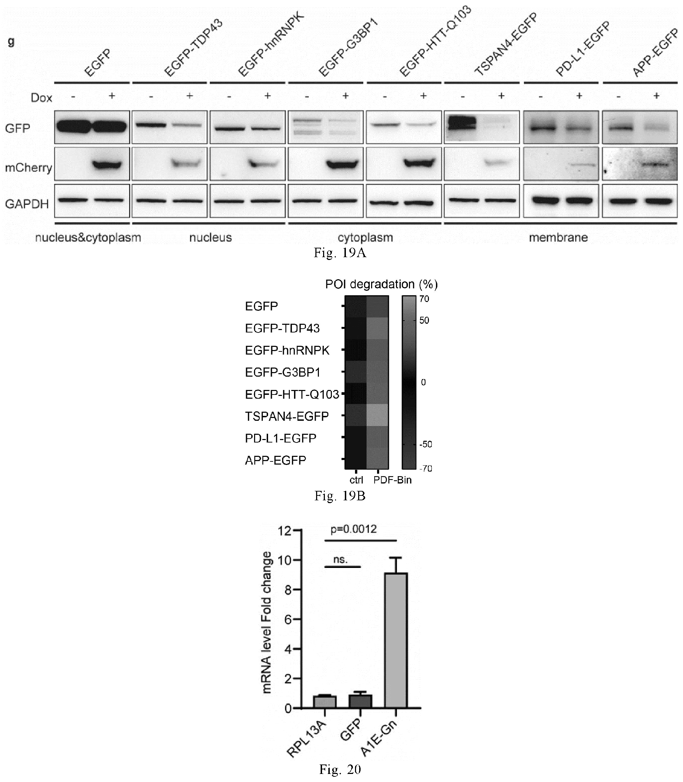

Fig. 19A illustrates Western blot results for the specified EGFP fusion proteins in HEK-293T cells that stably and inducibly express mCherry-A1E-Gn and were transfected with the specified constructs. - DOX indicates that the cells were treated with DMSO for 48 hours after transfection, and +DOX indicates that the cells were treated with 1 µg/mL DOX for 48 hours after transfection. A rabbit anti-GFP polyclonal antibody or rabbit anti-mCherry polyclonal antibody was used for detection, and GAPDH was used as an internal control.
Fig. 19B illustrates the statistical results of the gray values of the protein immunoblot bands in Fig. 19A. The horizontal axis represents the experimental conditions of the specified cell line, and the vertical axis represents the percentage of degradation of the specified construct. The statistical results shown are the average of three independent experiments, and the percentage of degradation is displayed by the corresponding gradient colors.
Fig. 20 illustrates the mRNA levels of specified genes, as detected by quantitative real-time PCR, in U-2OS cells that stably express EGFP-TDP43 and stably and inducibly express mCherry-A1E-Gn. RPL13A protein was used as an internal control. The horizontal axis represents the specified genes, and the vertical axis represents the fold change in mRNA level for each specified gene.

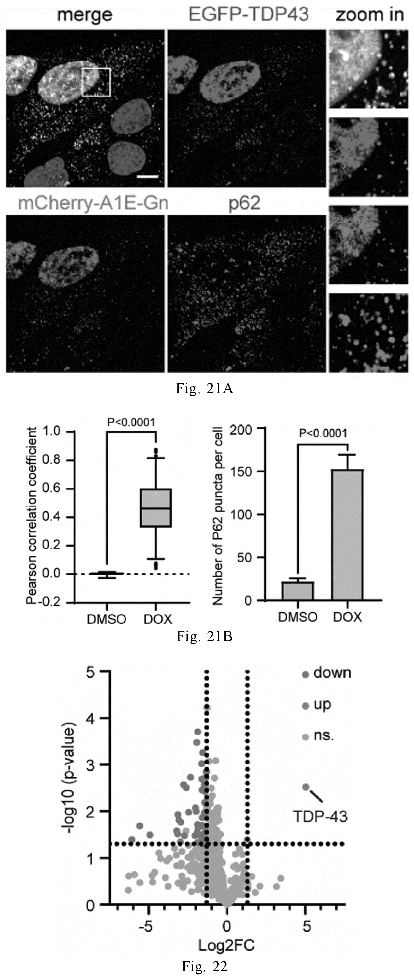

Fig. 21A shows laser confocal microscopy snapshots of U-2OS cells that stably express EGFP-TDP43 and stably and inducibly express mCherry-A1E-Gn after treatment for 24 hours with 1 µg/mL DOX (DOX+) or an equal volume of DMSO (DOX-). EGFP-TDP43, mCherry-A1E-Gn, and p62 respectively show fluorescence images of exogenous EGFP-TDP43, exogenous mCherry-A1E-Gn, and endogenous p62. Merge is the result obtained by overlaying the three images with the nuclear image (blue) in situ. The scale bar in each inset is 10 µm. Zoom in is an enlarged view of the white-boxed region on the left.
Fig. 21B illustrates the colocalization statistics obtained by Pearson colocalization analysis of the images in Fig. 21A (left) and the p62-body counts (right). The horizontal axis represents the specified treatment conditions, and the vertical axes represent the Pearson colocalization coefficient calculated for the corresponding condition (left) and the number of p62 bodies (right). The statistics shown are the mean ± standard error of the mean from three independent experiments, and p-values were calculated by a two-tailed t-test.
Fig. 22 illustrates the results of immunoprecipitation and mass spectrometry analysis of U-2OS cells stably expressing EGFP-TDP43 and stably induced to express mCherry-A1E-Gn after treatment with 1 µg/mL DOX or an equal volume of DMSO for 48 hours. The horizontal axis represents the fold change of the detected protein after DOX treatment, and the vertical axis represents the p-value of the fold change (FC). Red data points indicate high enrichment (log2FC>1.3, p<0.05), blue data points indicate low enrichment (log2FC<1.3, p<0.05), and gray data points indicate no difference (p>0.05).

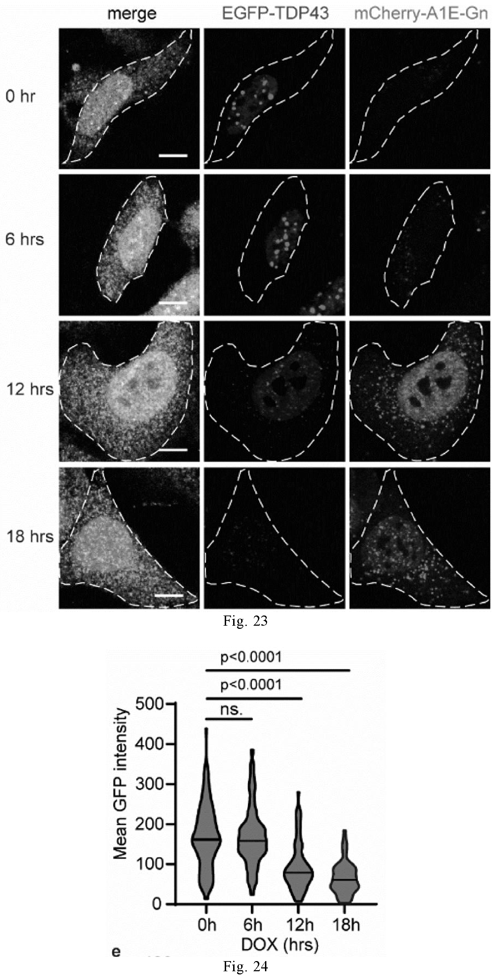

Fig. 23 illustrates laser confocal microscopy snapshots of U-2OS cells stably expressing EGFP-TDP43 and stably induced to express mCherry-A1E-Gn after treatment with 1 µg/mL DOX for a specified duration. The images show the fluorescence excitation of exogenous EGFP-TDP43 and mCherry-A1E-Gn, respectively. The Merge image is the result of overlaying these two images with the cell nucleus (blue) and β-actin (purple) in situ. The scale bar in each inset is 10 µm. White dashed lines indicate cell outlines.
Fig. 24 illustrates statistics obtained by fluorescence-intensity analysis of the images in Fig. 23. The horizontal axis represents the specified DOX treatment duration, and the vertical axis represents the mean GFP fluorescence intensity calculated for the corresponding treatment condition. The statistics shown are the mean ± standard error of the mean from three independent experiments, and the p-value was calculated by a two-tailed t-test. ns. indicates p > 0.05;

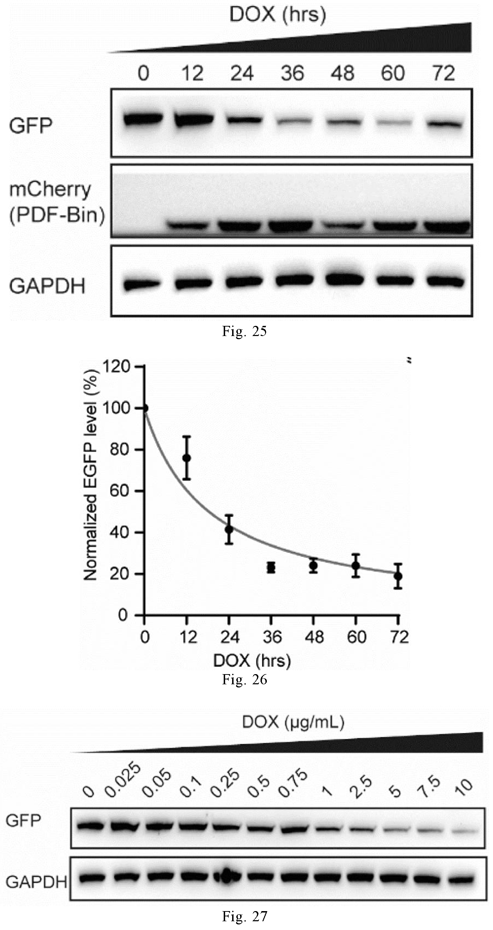

Fig. 25 illustrates Western blot bands from U-2OS cells that stably express EGFP-TDP43 and stably and inducibly express mCherry-A1E-Gn after treatment with 1 µg/mL DOX for the specified durations. EGFP-TDP43 was detected using a rabbit anti-GFP polyclonal antibody, mCherry-A1E-Gn was detected using a rabbit anti-mCherry polyclonal antibody, and GAPDH was used as an internal control;
Fig. 26 illustrates statistics for the grayscale intensities of the Western blot bands in Fig. 25. The horizontal axis represents the specified treatment duration, and the vertical axis represents the ratio of the grayscale intensity of the EGFP-TDP43 band at the specified time point to that at the start of treatment (0 hours). All grayscale intensities were normalized to the internal-control band. The statistics shown are the mean ± standard error of the mean from three independent experiments. P-values were calculated by a two-tailed t-test, and the data are presented as a nonlinear fit.
Fig. 27 illustrates Western blot bands from U-2OS cells that stably express EGFP-TDP43 and stably and inducibly express mCherry-A1E-Gn after treatment for 48 hours with the specified concentrations of DOX. EGFP-TDP43 was detected using a rabbit anti-GFP polyclonal antibody, and GAPDH was used as an internal control;

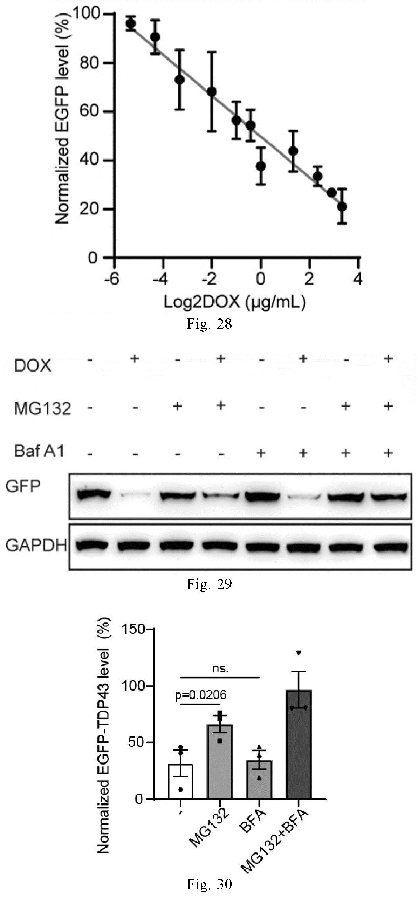

Fig. 28 illustrates statistics for the grayscale intensities of the Western blot bands in Fig. 27. The horizontal axis represents the specified treatment time, and the vertical axis represents the ratio of the grayscale intensity of the EGFP-TDP43 band at the specified time point to that at the start of treatment (0 hours). All grayscale intensities were normalized to the internal-control band. The statistics shown are the mean ± standard error of the mean from three independent experiments. P-values were calculated by a two-tailed t-test, and the data are presented as a linear fit.
Fig. 29 illustrates Western blot bands from U-2OS cells that stably express EGFP-TDP43 and stably and inducibly express mCherry-A1E-Gn after treatment with the inhibitor MG-132 or bafilomycin A1 (Baf A1). EGFP-TDP43 was detected using a rabbit anti-GFP polyclonal antibody, and GAPDH was used as an internal control;
Fig. 30 illustrates the statistical results of the gray values of the protein immunoblot bands in Fig. 29. The horizontal axis represents the specified treatment conditions, and the vertical axis represents the ratio of the gray value of the EGFP-TDP43 band under the specified treatment conditions to the gray value of the band without DOX treatment. All gray values were normalized using the gray value of the internal control band. The statistical results shown are the mean ± standard error of the mean of three independent experiments, and the p-value was calculated using a two-tailed t-test.

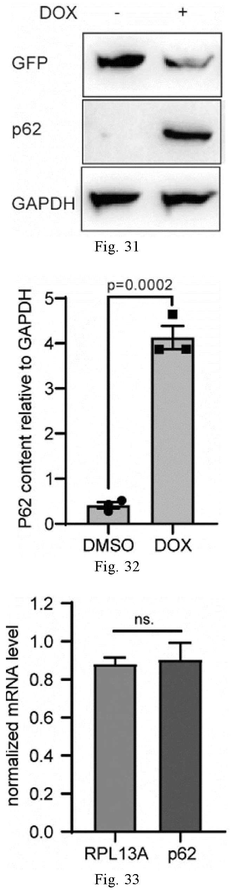

Fig. 31 illustrates the results of Western blot analysis of U-2OS cells stably expressing EGFP-TDP43 and stably induced to express mCherry-A1E-Gn after treatment with 1 µg/mL DOX (DOX+) or an equal volume of DMSO (DOX-) for 24 hours; anti-p62 rabbit polyclonal antibody was used for labeling, and GAPDH protein was used as an internal control.
Fig. 32 illustrates the statistical results of the gray values of the protein immunoblot bands in Fig. 31; the horizontal axis represents the specified treatment time, and the vertical axis represents the gray value of the p62 band under the specified treatment conditions. All gray values were normalized by the gray value of the internal control band; the statistical results shown are the mean ± standard error of the mean of three independent experiments, and the p value was calculated by a two-tailed t-test.
Fig. 33 illustrates the mRNA transcription levels of designated genes in U-2OS cells stably expressing EGFP-TDP43 and stably induced to express mCherry-A1E-Gn, as detected by quantitative real-time PCR. RPL13A protein was used as an internal control. The horizontal axis represents the designated genes, and the vertical axis represents the degree of mRNA variation of the designated genes.
   The left panel of Fig. 34 schematically shows a PDF-Bin targeting STAT3, and the right panel shows A549 cells stably and inducibly expressing EGFP-tagged A1E-MS3-6 (A549/A1E-MS3-6^{TRE}).
Fig. 35 illustrates laser confocal microscopy snapshots of A549 cells stably and inducibly expressing EGFP-A1E-MS3-6 after treatment for 24 hours with 1 µg/mL DOX (DOX+) or an equal volume of DMSO (DOX-). EGFP-A1E-MS3-6, STAT3, and p62 respectively show fluorescence images of exogenous EGFP-A1E-MS3-6, endogenous STAT3, and endogenous p62. Merge is the result obtained by overlaying the three images with the nuclear image (blue) in situ. The scale bar in each inset is 10 µm.
Fig. 36 illustrates the colocalization statistics obtained by enrichment analysis of mCherry fluorescence intensity in the images in Fig. 35. The horizontal axis represents the specified treatment conditions, and the vertical axis represents the enrichment of the mCherry fluorescence signal calculated for the corresponding treatment condition. The statistics shown are the mean ± standard error of the mean from three independent experiments, and the p-value was calculated by a two-tailed t-test.
Fig. 36 illustrates statistics for the number of p62 puncta in the images in Fig. 35. The horizontal axis represents the specified treatment conditions, and the vertical axis represents the mean number of p62 puncta per cell calculated for the corresponding treatment condition. The statistics shown are the mean ± standard error of the mean from three independent experiments, and the p-value was calculated by a two-tailed t-test.

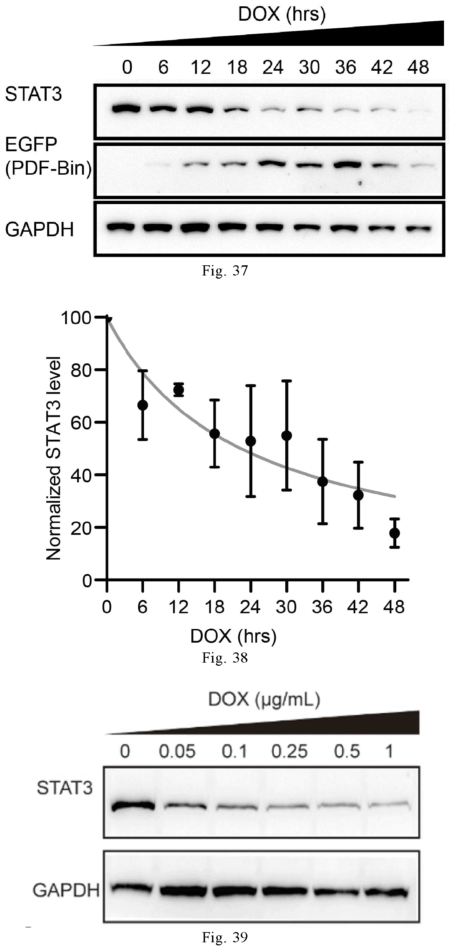

Fig. 37 illustrates Western blot bands from A549 cells stably and inducibly expressing EGFP-A1E-MS3-6 after treatment with 1 µg/mL DOX for the specified durations. STAT3 was detected using a rabbit anti-STAT3 polyclonal antibody, EGFP-A1E-MS3-6 was detected using a rabbit anti-GFP polyclonal antibody, and GAPDH was used as an internal control;
Fig. 38 illustrates statistics for the grayscale intensities of the Western blot bands in Fig. 37. The horizontal axis represents the specified treatment duration, and the vertical axis represents the ratio of the grayscale intensity of the STAT3 band at the specified time point to that at the start of treatment (0 hours). All grayscale intensities were normalized to the internal-control band. The statistics shown are the mean ± standard error of the mean from three independent experiments. P-values were calculated by a two-tailed t-test, and the data are presented as a nonlinear fit.
Fig. 39 illustrates Western blot bands from A549 cells stably and inducibly expressing EGFP-A1E-MS3-6 after treatment for 48 hours with the specified concentrations of DOX. STAT3 was detected using a rabbit anti-STAT3 polyclonal antibody, and GAPDH was used as an internal control;

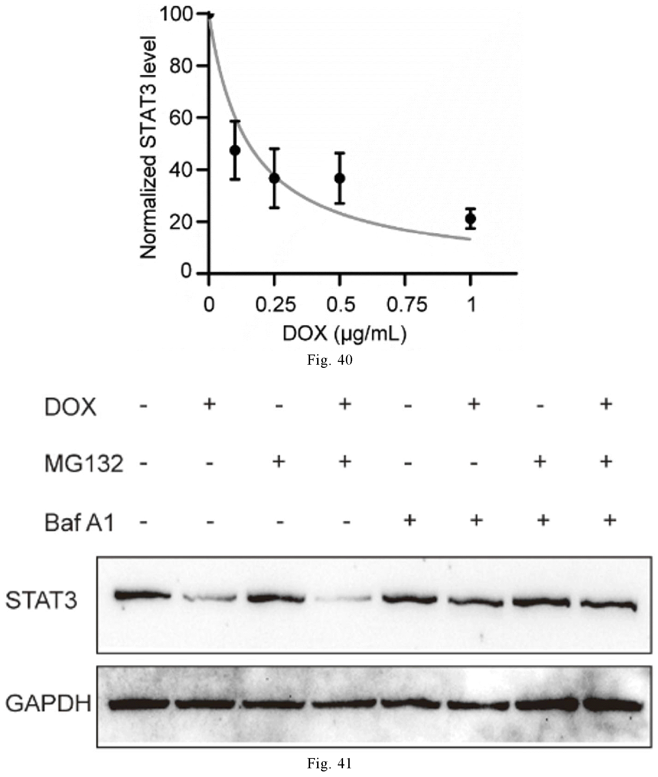

Fig. 40 illustrates statistics for the grayscale intensities of the Western blot bands in Fig. 39. The horizontal axis represents the specified treatment duration, and the vertical axis represents the ratio of the grayscale intensity of the STAT3 band at the specified time point to that at the start of treatment (0 hours). All grayscale intensities were normalized to the internal-control band. The statistics shown are the mean ± standard error of the mean from three independent experiments. P-values were calculated by a two-tailed t-test, and the data are presented as a linear fit.
Fig. 41 illustrates Western blot bands from A549 cells stably and inducibly expressing EGFP-A1E-MS3-6 after treatment with the inhibitor MG-132 or Baf A1. STAT3 was detected using a rabbit anti-STAT3 polyclonal antibody, and GAPDH was used as an internal control;

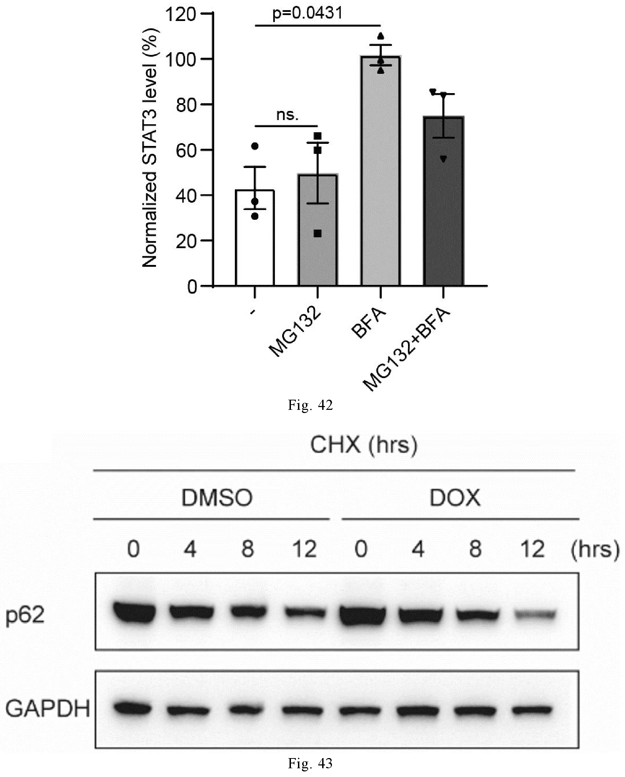

Fig. 42 illustrates the statistical results of the gray values of the protein immunoblot bands in Fig. 41. The horizontal axis represents the specified treatment conditions, and the vertical axis represents the ratio of the gray value of the STAT3 band under the specified treatment conditions to the gray value of the band without DOX treatment. All gray values were normalized using the gray value of the internal control band. The statistical results shown are the mean ± standard error of the mean of three independent experiments, and the p-value was calculated using a two-tailed t-test.
Fig. 43 illustrates Western blot bands from A549 cells stably and inducibly expressing EGFP-A1E-MS3-6 after treatment with cycloheximide (CHX) for the specified durations. p62 was detected using a rabbit anti-p62 polyclonal antibody, and GAPDH was used as an internal control;

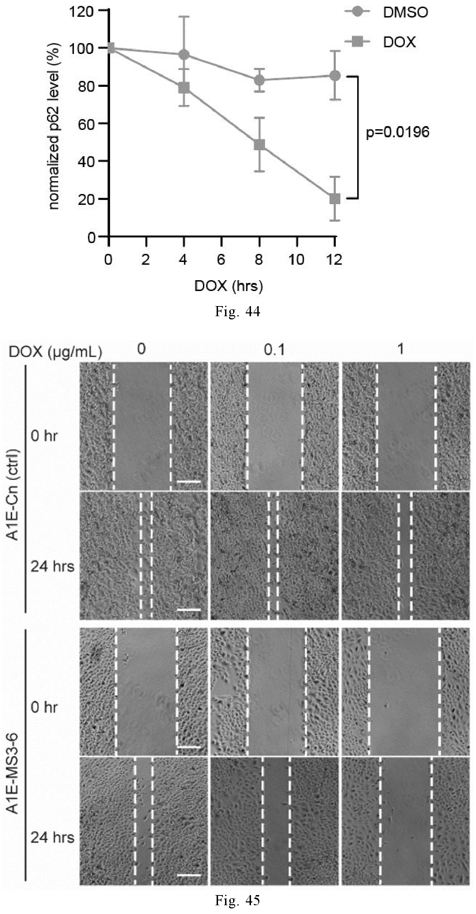

Fig. 44 illustrates the statistical results of the gray values of the protein immunoblot bands in Fig. 43; the horizontal axis represents the specified treatment time, and the vertical axis represents the gray value of the p62 band under the specified treatment conditions. All gray values were normalized by the gray value of the internal control band.
Fig. 45 illustrates microscopy snapshots from a scratch migration assay performed using A549 cells stably and inducibly expressing EGFP-A1E-MS3-6. The scale bar in each inset is 100 µm. White dashed lines indicate the edges of the scratched region;

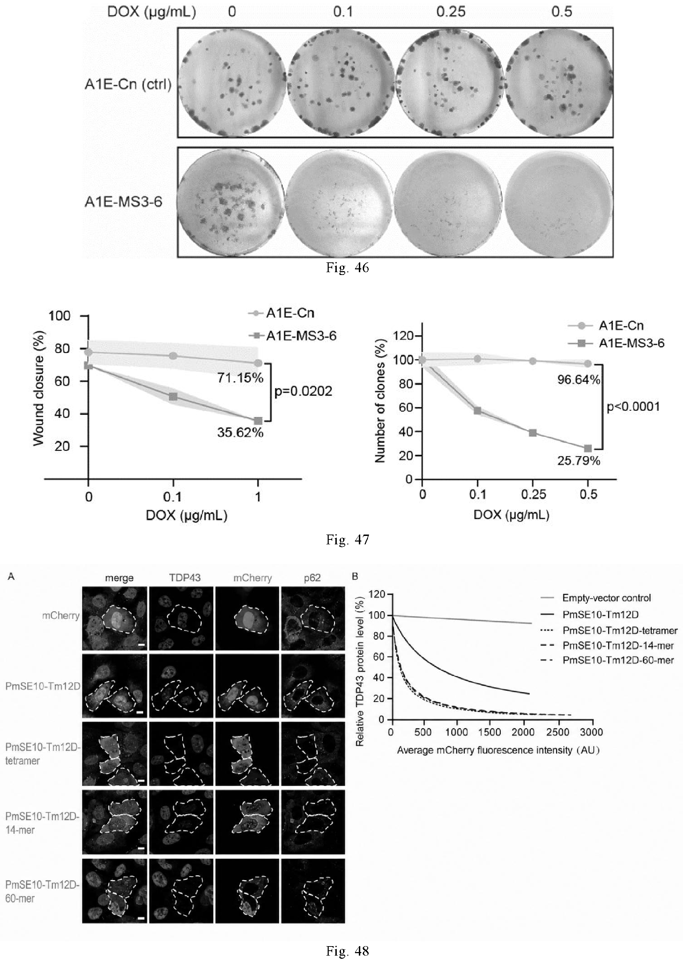

Fig. 46 illustrates microscopy snapshots from a colony formation assay performed using A549 cells stably and inducibly expressing EGFP-A1E-MS3-6. Purple spots indicate cell colonies;
Fig. 47 illustrates the statistical results for the degree of scratch healing in Fig. 45 (left) and the number of clones formed in Fig. 46 (right); the horizontal axis in the figure represents the specified treatment conditions, and the statistical results shown are the mean ± standard error of the mean of three independent experiments.
Fig. 48A illustrates the effect of oligomerization elements having different degrees of oligomerization on enhancing endogenous TDP43 degradation induced by a PDF-Bin molecule (PmSE10-Tm12D, which uses monobodies PmSE10 and Tm12D as binding arms). White dashed outlines indicate cells expressing the mCherry fusion protein, green indicates endogenous TDP43 protein, red indicates the PmSE10-Tm12D fusion protein, and purple indicates endogenous p62 protein. Fig. 48B shows the results of statistical analysis of fluorescence in Fig. 48A. The X-axis represents the average fluorescence intensity of the mCherry channel, indicating the expression level of the mCherry fusion protein; the Y-axis represents the percentage of TDP43 fluorescence intensity in transfected cells compared to untransfected cells in the same field of view, indicating the relative content of endogenous TDP43 protein; the results are presented using nonlinear fitting.

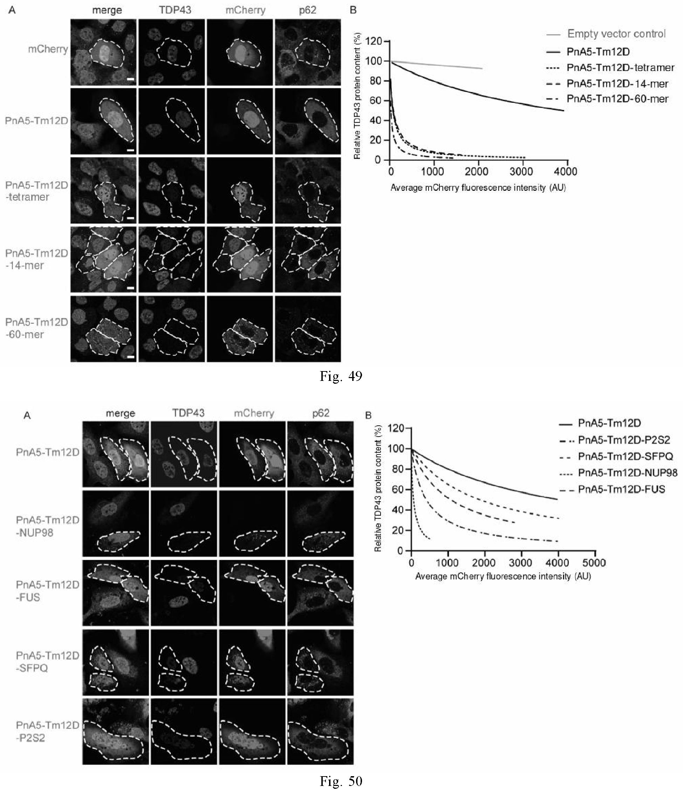

Fig. 49A illustrates the effect of oligomerization elements having different degrees of oligomerization on enhancing endogenous TDP43 degradation induced by another PDF-Bin molecule (PnA5-Tm12D, which uses nanobody PnA5 and monobody Tm12D as binding arms). Fig. 49B shows the results of statistical analysis of fluorescence in Fig. 49A. The presentation and analysis are the same as in Fig. 48.
Fig. 50A illustrates the effect of different types of phase-separation elements on enhancing endogenous TDP43 degradation induced by PnA5-Tm12D. Fig. 50B shows the results of statistical analysis of fluorescence in Fig. 50A. The presentation and analysis are the same as in Fig. 48.

### Detailed Description of Embodiments

The present invention is described in further detail below with reference to specific embodiments. The examples provided are intended only to illustrate the present invention and not to limit its scope. The examples provided below are intended as guidance for further improvements by a person of ordinary skill in the art and do not limit the present invention in any way.

Unless otherwise specified, the experimental methods used in the following examples are conventional methods performed according to techniques or conditions described in the literature in the art or according to the product instructions. Unless otherwise specified, all materials, reagents, instruments, etc. used in the following examples are commercially available. Unless otherwise specified, quantitative experiments in the following examples are reported as the mean of three replicate experiments. Unless otherwise specified, in the following examples, each nucleotide sequence in the sequence listing is written from left to right in the 5'-to-3' direction, and each amino acid sequence is written from left to right from the amino terminus to the carboxyl terminus.

### Definitions

As used in the specification of this invention, the following words and phrases are generally considered to have the meanings set forth below, unless otherwise specified in the context in which they are used.

As used herein, the singular forms "a," "an," and "the" refer to both the singular and the plural, unless the context clearly indicates otherwise. As used herein, the terms "comprising," "including," and "having" mean that a composition or method includes the recited components or steps but does not exclude other components or steps.

As used herein, the term "about" refers to the common range of error for the corresponding value that is readily known to those skilled in the art. For example, within ±10%, within ±5%, within ±2%, within ±1%, or within ±0.1% of the stated value. A value or parameter described herein using "about" includes the value or parameter itself.

As used herein, the term "liquid-liquid phase separation" (LLPS, also referred to herein as "phase separation" or "phase transition") refers to the following transition process occurring among multivalent macromolecules: under suitable solution conditions, the multivalent macromolecules aggregate through interactions to form larger complexes, which reach their corresponding solubility limit and separate from the bulk solution phase to form an independent liquid phase enriched in the complexes. Biological macromolecules can aggregate and undergo phase separation due to intermolecular or intramolecular interactions. Modules or motifs capable of causing the foregoing intermolecular or intramolecular interactions include, but are not limited to: (1) linearly arranged structural modules or motifs having similar functions in a protein or polypeptide; (2) structural modules or motifs that promote oligomerization of a protein or polypeptide; (3) polimerization binding sites generated through post-translational modifications; and (4) intrinsically disordered regions or low-complexity domains in a protein or polypeptide (see, for example, Wang et al., Cell 174(3): 688-699, 2018; Nott, Timothy J et al., Molecular Cell 57(5): 936-947, 2015).

The term "phase-transition droplet" refers to a readily recognizable small droplet, having a diameter of up to several micrometers or even larger, that is present in a liquid phase formed by phase separation. Herein, a "phase-transition droplet" is sometimes also referred to simply as a "droplet."

As used herein, the terms "oligomerization," "condensation," and "aggregation," when describing biomacromolecules such as proteins, mean that several biomacromolecules, such as several receptor molecules, associate through noncovalent interactions to form a complex, potentially changing their functional state.

The term "intact antibody" refers to an antibody having a structure substantially similar to that of a naturally occurring antibody. Herein, "intact antibody" may be used interchangeably with "full-length antibody" and "whole antibody."

The term "antigen-binding fragment," or an equivalent expression, refers to a molecule other than an intact antibody that comprises a portion of the intact antibody, which portion binds an antigen that can be bound by the intact antibody. Examples of antigen-binding fragments include, but are not limited to, bis-Fab, Fv, Fab, Fab, Fab'-SH, F(ab')₂, and the like.

As used herein, the term "nanobody" refers to a form of antibody or antigen-binding fragment that is much smaller than a conventional four-chain antibody. Most known nanobodies are derived from the variable region (V_{H}) of an antibody heavy chain, typically comprise approximately 120 amino acids, have a molecular weight of approximately 12-15 kDa, and measure approximately 4 × 2.5 nm. This type of antibody was first reported under the concept of a "single-domain antibody" in 1989, when Ward, E. et al. found that two murine V_{H} domains screened from a cDNA expression library prepared from the spleens of mice immunized with lysozyme and keyhole limpet hemocyanin exhibited affinity for lysozyme (see Ward, E. et al., Nature 341, 544-546 (1989)). Subsequently, large quantities of naturally occurring IgG comprising only heavy chains (HCAb) were found in camelid serum, accounting for 45% to 75% of total serum immunoglobulin, depending on the species. HCAb naturally lacks the light chains found in conventional four-chain antibodies and consists of two heavy chains, each comprising one heavy-chain variable region (V_{HH}), one hinge region, and two domains, CH₂ and CH₃; it is referred to as a "heavy-chain-only antibody" or "heavy-chain antibody." Similar naturally occurring heavy-chain antibodies are also found in cartilaginous fish (V_{NAR}) (Feng et al., Antib Ther, 2, 1-11, 2019) and in certain human heavy-chain diseases (Prelli and Frangione, J Immunol, 148, 949-952, 1992). Recombinant nanobodies based on camelid V_{HH} domains are currently the principal focus of nanobody research and development. Compared with human V_{H} domains, the following structural features give naturally evolved camelid V_{HH} domains superior solubility and stability: according to Kabat numbering, V37 in the human V_{H} germline is typically F37 or Y37 in V_{HH} domains, resulting in more compact and stable packing of the hydrophobic core (Riechmann and Muyldermans, J Immunol Methods, 231, 25-38, 1999; Shinozaki et al., J Biosci Bioeng, 125, 654-661, 2018); the light-chain-contact residues G44, L45, and W47 in the human V_{H} germline correspond to E44 (or Q44), R45 (or C45), and G47 (or Ser, Leu, or Phe), respectively, in V_{HH} domains (Holt et al., Trends Biotechnol, 21, 484-490, 2003), making the accessible surface area more hydrophilic and less prone to aggregation; in some V_{HH} domains, W103 may be replaced with R103; V_{HH} domains typically have a longer CDR3 than human/rodent V_{H} domains, and their CDR3 generally contains Cys, which can form, with a Cys at the end of CDR1 (camel) or the beginning of CDR2 (llama), an additional disulfide bond besides the canonical C22-C92 disulfide bond (Wesolowsk et al., Med Microbiol Immunol, 198, 157-174, 2009), thereby making the V_{HH} domain more stable (Tm values ranging from 60-78°C) and enabling reversible unfolding/refolding (Holt et al., Trends Biotechnol, 21, 484-490, 2003).

The term "single-chain antibody," also known as "single-chain Fv," "single-chain variable fragment," "sFv," or "scFv," refers to an antibody fragment comprising V_{H} and V_{L} antibody domains linked in a single polypeptide chain. Preferably, the scFv polypeptide further includes a polypeptide linker between the V_{H} and V_{L} domains, enabling the scFv to form the desired antigen-binding structure. For a review of scFv, see Pluckthun, The Pharmacology of Monoclonal Antibodies, Vol. 113, edited by Rosenburg and Moore, Springer Verlag, New York, pp. 269-315 (1994); Malmborg et al., J. Immunol. Methods 183:7-13, 1995.

The term "single-domain antibody" refers to an antibody fragment that contains all or part of the heavy chain variable domain or all or part of the light chain variable domain. In some aspects, the single-domain antibody is a human single-domain antibody (see, for example, U.S. Patent No. 6,248,516B1). Examples of single-domain antibodies include, but are not limited to, V_{HH}.

The term "complementarity-determining region" or "CDR" refers to a region of an antibody variable region that is primarily responsible for binding to an antigen epitope. CDRs may be located and aligned using the three-dimensional space formed by antibody framework regions, and the amino acid sequence boundaries of a given CDR can be readily determined using any of various well-known numbering schemes, such as Kabat et al. (see Sequences of Proteins of Immunological Interest, U.S. Department of Health and Human Services, 1991; the "Kabat" numbering scheme), Chothia et al. (see Chothia and Lesk, J Mol Biol 196:901-917, 1987; Chothia et al., Nature 342:877, 1989; and Al-Lazikani et al., JMB 273, 927-948, 1997; the "Chothia" numbering scheme), Kunik et al. (see Kunik et al., PLoS Comput Biol 8:el002388, 2012; and Kunik et al., Nucleic Acids Res 40 (Web Server issue): W521-W524, 2012; "Paratome CDRs"), and the ImMunoGeneTics (IMGT) database (see Lefranc, Nucleic Acids Res 29:207-209, 2001; the "IMGT" numbering scheme). Kabat, Paratome, and IMGT are all publicly available databases on the Internet.

The term "small molecule" refers to any molecule with a molecular weight of about 2,000 Daltons or less, such as about 1,000 Daltons or less. In some aspects, a small molecule may be an organic molecule. In other aspects, a small molecule may be an inorganic molecule.

As used herein, "p62 protein" is a component of inclusion bodies found in protein-aggregation diseases of the brain and liver. It was identified as having a molecular weight of 62 kDa and binding, in a phosphotyrosine-independent manner, to the Src homology 2 (SH2) domain of the tyrosine kinase Lckp56 (Park et al., Proc Natl Acad Sci USA, 92:12338 (1995)). The term "p62" or "p62 protein" broadly refers to any naturally occurring p62 molecule from any vertebrate source, including mammals such as primates (e.g., humans), non-human primates (e.g., cynomolgus monkeys), and rodents (e.g., mice and rats). The term encompasses full-length p62 and isolated regions or domains of p62. The term also covers naturally occurring variants of p62, such as splice variants or allele variants. An exemplary human p62 DNA sequence is set forth in SEQ ID NO 8, and an exemplary human p62 amino acid sequence is set forth in SEQ ID NO 7. The present invention also contemplates minor sequence variations, particularly conservative amino acid substitution variants that do not affect p62 function and/or activity. p62 has been reported to exhibit ubiquitin-binding activity in vivo (Vadlamudi et al., J. Biol. Chem., 271: 20235 (1996)). Furthermore, p62 is homologous to neither ubiquitin C-terminal hydrolases nor the S5a subunit of the 26S proteasome complex (the only protein known to bind ubiquitin noncovalently), indicating that p62 belongs to a novel class of ubiquitin-binding proteins. It has been found that protein aggregates containing p62 can be degraded through autophagy. It has been proposed that this function of p62 may protect against huntingtin-induced cell death (Bjørkøy et al., J Cell Biol., 171:603 (2005)).

The term "intrinsically disordered protein" (also referred to herein as a "disordered protein") refers to a class of proteins/polypeptide fragments that lack a stable, ordered secondary and/or tertiary structure under physiological conditions, are fully or partially unfolded in their native state, but are capable of performing normal biological functions. The term "intrinsically disordered region" (also referred to as "disordered region" herein) refers to a region that does not fold into a fixed three-dimensional structure under physiological conditions, but exists in a heterogeneous set of conformations. Intrinsically disordered proteins are widespread in organisms. Because disordered proteins and disordered regions are highly flexible and lack stable secondary/tertiary structures, they are not limited to interacting with a single target and generally play important roles in cellular signal transduction and protein interaction networks. Naturally occurring intrinsically disordered proteins are known in the art from the literature or databases. For example, the DisProt database compiles intrinsically disordered proteins selected from the literature (Hatos, A. et al., DisProt: Intrinsic protein disorder annotation in 2020, Nucleic Acids Research (2020) 48 (D1) 269-276). Many polypeptides and proteins associated with various diseases (including cancer, cardiovascular disease and neurodegenerative diseases) are known to have disordered structures, including but not limited to important regulatory factors such as p53 and c-Myc. Disordered structures often exhibit a preference in amino acid composition, such as being rich in polar amino acids like G, P, E, S, Q, K, D, T, and R, as well as aromatic amino acids like Y and F.

The term "oligomerization element" refers to an amino acid sequence that forms a structure capable of interacting with an oligomerization element (identical or different) in another polypeptide, thereby enabling the polypeptides to form oligomers through noncovalent interactions. Common oligomerization elements are oligomerization domains from proteins that naturally form oligomers (homo-oligomers or hetero-oligomers), such as associations between β-sheets, α-helices, or hydrophobic surface patches in different monomers. A common motif for protein oligomerization is the coiled-coil domain. An α-helical structural motif can itself form a helix, and two, three, four, or five α-helices can intertwine to form a left-handed supercoil known as a "coiled coil." The simplicity of coiled-coil domains makes them a conventional choice for designing fusion proteins having a defined oligomerization state. Hundreds of coiled-coil domain sequences are known in the art; see, for example, Arai, R. (2021). Design of helical linkers for fusion proteins and protein-based nanostructures. Methods Enzymol 647, 209-230; Bozic, S., Doles, T., Gradisar, H., and Jerala, R. (2013). New designed protein assemblies. Curr Opin Chem Biol 17, 940-945; Dawson, W.M., Martin, F.J.O., Rhys, G.G., Shelley, K.L., Brady, R.L., and Woolfson, D.N. (2021). Coiled coils 9-to-5: rational de novo design of α-helical barrels with tunable oligomeric states. Chemical Science 12, 6923-6928; Fletcher, J.M., Boyle, A.L., Bruning, M., Bartlett, G.J., Vincent, T.L., Zaccai, N.R., Armstrong, C.T., Bromley, E.H.C., Booth, P.J., Brady, R.L., et al. (2012). A Basis Set of de Novo Coiled-Coil Peptide Oligomers for Rational Protein Design and Synthetic Biology. ACS Synthetic Biology 1, 240-250; Fletcher, J.M., Harniman, R.L., Barnes, F.R.H., Boyle, A.L., Collins, A., Mantell, J., Sharp, T.H., Antognozzi, M., Booth, P.J., Linden, N., et al. (2013). Self-Assembling Cages from Coiled-Coil Peptide Modules. Science 340, 595-599; Hsia, Y., Bale, J.B., Gonen, S., Shi, D., Sheffler, W., Fong, K.K., Nattermann, U., Xu, C., Huang, P.S., Ravichandran, R., et al. (2016). Design of a hyperstable 60-subunit protein dodecahedron. [corrected]. Nature 535, 136-139, among others. Any suitable sequence that can oligomerize with other coiled-coil domains without disrupting the antigen-binding function of the nanobody can be used as an oligomerization element in this invention. As a non-limiting example of an oligomerization domain, an α-helix having an abcdefg heptad repeat may be used; the repeat forms a nonpolar stripe of interacting hydrophobic residues on one side of each helix, and electrostatic interactions may also occur between side chains on opposite sides of the helix. The nonpolar stripe is defined by hydrophobic side chains at positions a and d; Leu, Ile, or Ala is most common at position a, and Leu or Ala is typical at position d. Electrostatic interactions mainly occur at residues e and g, which are typically Glu or Gln. Arg and Lys are also mainly at position g. Charged residues interact with the solvent and typically appear at positions b, c, and f. Another type of oligomerization domain is a left-handed triple helix known as a collagen helix, which comprises a basic 1Gly-2Xaa-3Xaa tripeptide repeat, wherein 2Xaa is typically Pro and 3Xaa is typically 4-hydroxyproline.

The term "phase-separation element" refers to an element comprising multiple structural modules or motifs associated with phase separation. Biological macromolecules can aggregate and undergo phase separation due to intermolecular or intramolecular interactions. Modules or motifs capable of causing the foregoing intermolecular or intramolecular interactions include, but are not limited to: (1) linearly arranged structural modules or motifs having similar functions in a protein or polypeptide; (2) structural modules or motifs that promote oligomerization of a protein or polypeptide; (3) multivalent binding sites generated through post-translational modifications; and (4) intrinsically disordered regions or low-complexity domains in a protein or polypeptide. See, for example, Wang et al., Cell 174(3): 688-699, 2018; Nott, Timothy J et al., Molecular Cell 57(5): 936-947, 2015. In some cases, the phase-separation element of the present invention is formed by tandemly linking one or more SUMO3 motifs and one or more SIM motifs. In other cases, the phase-separation element of the present invention is formed by tandemly linking one or more PRMH motifs and one or more SH3 motifs.

The terms "increase" and "activation" as used herein mean the ability to cause an overall increase, for example, an overall increase of 10% or more, 20% or more, 30% or more, 40% or more, 50% or more, or 75%, 85%, 90%, 95%, or more. In some aspects, an increase or activation may refer to downstream activity resulting from a ligand-cell-surface-molecule interaction.

The terms "decrease" and "inhibition" as used herein mean the ability to cause an overall decrease, for example, an overall decrease of 10% or more, 20% or more, 30% or more, 40% or more, 50% or more, or 75%, 85%, 90%, 95%, or more. In some aspects, a decrease or inhibition may refer to downstream activity resulting from a ligand-cell-surface-molecule interaction.

The preferred embodiments for carrying out the present invention will now be described. It should be noted that the embodiments described below are examples illustrating representative embodiments of the present invention, but the present invention is not limited to these examples.

Two or more of the embodiments described below may be combined, and such combinations are also encompassed by the present invention.

### Examples

The present invention is described in further detail below with reference to specific examples. The examples provided are intended only to illustrate the present invention and not to limit its scope. The examples provided below may serve as guidance for further improvements by those of ordinary skill in the art and do not limit the present invention in any way.

Unless otherwise specified, the experimental methods used in the following examples were conventional methods performed according to techniques or conditions described in the literature in the art or according to the product instructions. Unless otherwise specified, all materials, reagents, instruments, etc. used in the following examples are commercially available. The relevant nucleic acids, genes, and enzymes may also be obtained from information in public databases using conventional molecular biology techniques.

Unless otherwise specified, nucleotide sequences referred to herein are written from left to right in the 5'-to-3' direction, and amino acid sequences are written from left to right from the amino terminus to the carboxyl terminus. If a sequence set forth in the description is inconsistent with the sequence listing, the sequence set forth in the description shall prevail.

### Experimental Materials, Reagents, Instruments, and Methods

### Plasmid construction:

The plasmids used herein may all be constructed from commercially available original plasmids using conventional gene cloning and recombination methods in the field (see, for example, "Molecular Cloning: A Laboratory Manual (3rd Edition)" (Science Press) and "Microbiology Experiments (4th Edition)" (Higher Education Press)). All coding gene sequences involved could be obtained from information in public databases using conventional molecular biology techniques and were synthesized under contract by Xianghong Biotechnology. The seamless cloning kit used was obtained from Beyotime (D7010M), the competent cells were Stbl3 cells (Kangti Life, KTSM110L), and ampicillincontaining LB agar plates were used as the selection medium.

### (1) pcDNA3.1-mCherry-4xGGS-Xho I-Apa I

pcDNA3.1 (Invitrogen, V79020) was used as the initial backbone plasmid to construct a eukaryotic expression vector, and its ampicillin-resistance marker was used for selection and amplification in Escherichia coli. The coding sequence of the mCherry fluorescent tag (SEQ ID NO 1), the 4xGGS flexible linking sequence (SEQ ID NO 2), and the Xho I and Apa I restriction sites for insertion of a gene of interest were inserted between the BamH I and Xho I restriction sites. The mCherry fluorescent tag (red) may be used to visualize and track an antibody or PDF-Bin fused thereto.

### (2) pcDNA3.1-EGFP-4xGGS-Xho I-Xba I

The construction method was substantially the same as that in (1), except that the coding sequence of the EGFP fluorescent tag (SEQ ID NO 15) was inserted and Xho I and Xba I were used as the restriction sites. The EGFP fluorescent tag (green) may be used to visualize and track p62 and variants thereof.

### (3) pcDNA3.1-EGFP-NUP98N-Xho I-Xba I

The construction method was substantially the same as that in (2), except that the 4xGGS linking sequence was replaced with the coding sequence of the N-terminal disordered domain of NUP98 (NUP98N) (SEQ ID NO 4).

### (4) pLVX-EGFP-TDP43

pLVX (Takara, 632164) was used as the initial backbone plasmid to construct a lentiviral expression vector that can be used to package lentiviral particles, generate stable cell lines, and assess expression of a protein of interest by transient transfection. The plasmid provides ampicillin resistance in prokaryotic cells and puromycin resistance in eukaryotic cells. A gene of interest may be inserted after the vector is linearized by PCR. The primers used to linearize the vector by PCR were as follows:
pLVX-F: ACCGGTTAACTCGAGCATGC (SEQ ID NO 5)
pLVX-R: GCGGCCGCAAGCTTGGGTCT (SEQ ID NO 6)

### (5) pLVX-TETONE-EcoR I-Age I

Using pLVX-TETONE (Takara, 631847) as the initial backbone plasmid, a lentiviral expression vector was constructed. This vector can be used to package lentiviruses, construct stable cell lines, and control the expression of target genes through the tet-on system inherent in the plasmid. The plasmid provides ampicillin resistance in prokaryotic cells and puromycin resistance in eukaryotic cells. The gene of interest was inserted into the vector site linearized with the restriction enzymes EcoR I and Age I.

### Cell lines:

HEK-293T: ATCC, CRL-3216;
U-2OS: ATCC, HTB-96;
A549: ATCC, CCL-185.

### Cell culture:

The HEK-293T, U-2OS, and A549 cell lines used in the examples were cultured in high-glucose DMEM (HyClone, SH30243.01) containing 10% fetal bovine serum (FBS; Gibco, 10099-141) and 1% penicillin-streptomycin solution (Gibco, 15140122). Cells were cultured in a humidified incubator at 37°C with 5% CO₂. When the cells reached 85% confluence, they were digested and passaged using trypsin. Depending on the screening requirements, puromycin or other corresponding antibiotics may be added to the culture medium to a final concentration of 1 µg/mL.

### Cell transfection:

Depending on the experimental requirements, cells were seeded in culture plates or dishes of different well sizes and materials and cultured. When the cells reached 50-80% confluence, they were transfected using the Lipo8000 Transfection Reagent (Beyotime, C0533) according to the manufacturer's instructions.

### Lentiviral infection:

All stable cell lines used herein were generated using lentivirus. The lentiviral packaging plasmids used were pMD2.G (Addgene, 12259) and psPAX2 (Addgene, 12260). The cells to be transduced were cultured to a suitable density (e.g., approximately 50% confluence). Before transfection, the culture medium was replaced with preheated antibiotic-free culture medium. Then, according to the manufacturer's instructions, the transfection reagent PEI (Polysciences, 23966-100), two packaging plasmids psPAX2 and pMD2.G, a lentiviral plasmid containing the target gene, and Opti-MEM medium were mixed and incubated at room temperature for 10 minutes to prepare the transfection mixture. The mixture was then added evenly and dropwise to the culture dish, and the cells were incubated for an additional 8-12 hours in a cell-culture incubator. The medium was replaced with fresh medium. Thereafter, the culture supernatant was collected every 24 hours and filtered through a 0.22-µm syringe filter, and the recombinant virus was added to the cells to be transduced (approximately 50% confluence). The virus-producing HEK-293T cells were cultured in fresh medium, and viral supernatant could be collected two to three consecutive times. Stable cells may be enriched using an appropriate method, such as flow cytometry or antibiotic selection. All stable cell lines used herein were monoclonal cell lines generated by single-cell sorting of stable cell populations by flow cytometry, followed by selection based on fluorescence intensity or inducible expression after expansion.

### Immunofluorescence staining:

Cells were seeded into 4-well glass culture dishes (In Vitro Scientific, # D35C4-20-1-N) and incubated for 12-18 hours. Cells were transfected with the specified vector or subjected to the specified treatment. After discarding the culture medium, the cells were gently rinsed with PBS buffer. Cells were fixed at room temperature for 15 minutes with 200 µL of 4% paraformaldehyde solution (Beyotime, P0099). The liquid was then aspirated, and the cells were gently rinsed three times with PBS buffer. Cells were permeabilized with permeabilization buffer (Beyotime, P0097) at room temperature for 15 minutes, after which the liquid was aspirated and the cells were gently rinsed three times with PBS. Cells were blocked at room temperature for 1 hour using immunofluorescence blocking solution/primary antibody diluent (Sangon Biotech, E674004), after which the liquid was aspirated. Cells were incubated with the specified primary antibody at room temperature for 1 hour, after which the liquid was aspirated and the cells were gently rinsed three times with PBS. Cells were incubated with the specified fluorophore-conjugated secondary antibody at room temperature in the dark for 1 hour, after which the liquid was aspirated and the cells were gently rinsed three times with PBS. Hoechst 33342 staining solution was diluted 1:2,000 with PBS and added to the cells, and the cells were incubated in the dark for 5-10 minutes. The liquid was aspirated, the cells were gently rinsed three times with PBS, and the cells were finally stored in PBS.

### Bio-Layer Interferometry (BLI):

The antibody to be measured was biotinylated and immobilized on an SA biosensor. Human p62 protein (SEQ ID NO 7) was diluted in assay buffer (1× PBS, pH 7.4, 0.05% Tween-20) to obtain p62 protein solutions at 5 nM, 15 nM, 40 nM, and 60 nM, and each solution was reacted with the immobilized antibody. The reaction process was detected using the Octet K2 protein analysis system (Sartorius, Octet K2) and streptavidin (SA) biosensor. The reaction data were normalized using Octet Data Analysis Studio 12.2 and plotted using GraphPad Prism 8.

### CoPIC, a cell-based protein-protein interaction system:

Reference may be made, for example, to the CoPIC method described in Xu, W. et al., Compartmentalization-aided interaction screening reveals extensive high-order complexes within the SARS-CoV-2 proteome. Cell Reports 36, 2021. Purified plasmids were co-transfected into U-2OS cells. After 24 hours of culture, the nuclei of live cells were stained with Hoechst 33342 (Thermo Scientific, 62249), and the cells were then observed and imaged using a Nikon laser confocal microscope. Nikon NIS image-analysis software was used to measure the mean fluorescence intensity of the mCherry signal inside and outside the EGFP droplets, and enrichment of the mCherry signal in the EGFP droplets was calculated as follows: $mCherry enrichment level = \left(I{\left(T\right)}_{inside droplet}/I{\left(T\right)}_{outside droplet}\right) - \left(I{\left(C\right)}_{inside droplet}/I{\left(C\right)}_{outside droplet}\right)$

### Western blotting:

Cell samples were lysed on ice for 10 minutes using low-salt lysis buffer (50 mM Tris-HCl, 2% Triton X-100, 2 mM EDTA, pH 7.4) to obtain total cellular protein. Next, 5× loading buffer (0.6 M Tris-HCl, pH 6.8, 2% SDS, 25% glycerol, 14.4 mM DTT, and 0.1% bromophenol blue) was added, and the samples were heated in a metal bath at 98°C for 10 minutes. An appropriate amount of each protein sample was loaded onto a precast gradient gel, and electrophoresis was performed at 110 V for approximately 70 minutes. Under constant current conditions of 150 mA for 60 minutes, the electrophoretic bands were transferred onto a PVDF membrane pre-activated with anhydrous methanol. After transfer, the membrane was blocked in TBST containing 5% nonfat dry milk at room temperature for approximately 1 hour. After rinsing the membrane three times with TBST solution, the membrane was placed in the primary antibody solution and incubated overnight at 4°C. Then, the PVDF membrane was rinsed three times with TBST solution, and then placed in a horseradish peroxidase (HRP)-conjugated secondary antibody solution and incubated on a shaker at room temperature for 1 hour. Each antibody solution used was diluted at the specified ratio in TBST containing 5% nonfat dry milk. After the PVDF membrane was rinsed three times with TBST, it was placed in an imaging instrument, developing solution was added evenly, and the signal was developed and imaged.

### Quantitative Real-Time PCR (RT-qPCR):

RNA was extracted using an M5 EASYspin Plus RNA Rapid Extraction Kit (MF159) according to the manufacturer's instructions. After the RNA concentration was measured using a NanoDrop instrument, the RNA was stored at -80°C. Total RNA was used as a template for reverse transcription using an M5 Super plus qPCR RT kit with gDNA remover (MF166-plus) according to the kit instructions. Specifically, a 10-µL reaction system containing 1 µg of RNA, 1 µL of 10× gDNA remover mix, and the remaining volume of DEPC-treated water was incubated at 42°C for 2 minutes and then cooled on ice. Next, 4 µL of 5× M5 RT Super plus and 6 µL of DEPC-treated water were added to the reaction solution, which was incubated at 37°C for 15 minutes, inactivated at 85°C for 5 seconds, and then cooled on ice to obtain reverse-transcribed cDNA. The resulting cDNA solution was diluted to 120 µL and used as a template for RT-qPCR using 2× M5 HiPer SYBR Premix EsTaq (with Tli RNaseH) (MF787). Specifically, the 10 µL reaction system contained 5 µL of 2x M5 HiPer SYBR Premix EsTaq (with Tli RNaseH), 1 µL of template cDNA, 0.5 µL each of forward and reverse primers, and 3 µL of DEPC water. RT-qPCR was performed on a Roche LightCycler^{®} 480 Instrument II using the primer pairs shown in Table 1.

**Table 1:**

| **Gene** | **Sequence (5'→3')** | **SEQ ID NO.** |
|---|---|---|
| RPL13A | F: GCCATCGTGGCTAAACAGGTA | 42 |
| | R: GTTGGTGTTCATCCGCTTGC | 43 |
| GFP | F: CTGAGCAAAGACCCCAACGA | 44 |
| | R: TCGTCCATGCCGAGAGTGAT | 45 |
| A1E-Gn | F: GCGGTGGTGGTTTTGTTCAG | 46 |
| | R: TTCCCAAGAGTAACCGCTGG | 47 |
| p62 | F: GACTACGACTTGTGTAGCGTC | 48 |
| | R: AGTGTCCGTGTTTCACCTTCC | 49 |

### Immunoprecipitation coupled with mass spectromery (IP-MS):

Immunoprecipitation was first performed. Cells were lysed at 4°C for 30 minutes in low-salt lysis buffer containing a protease-inhibitor cocktail and then centrifuged at 10,000 × g for 5 minutes. The supernatant was mixed with rabbit anti-p62 polyclonal antibody and protein A/G magnetic beads (bimake, #B23201) and incubated overnight at 4°C. The magnetic beads were thoroughly washed five times with low-salt lysis buffer, and the immunoprecipitates were eluted with 2× SDS loading buffer and analyzed by SDS-PAGE.

Mass spectrometry was then performed. The target-protein band was excised from the SDS-PAGE gel, incubated with 25 mM chloroacetamide at 55°C for 45 minutes in the dark for alkylation, and then digested with trypsin at 37°C for 14 hours. The peptides were extracted three times with 50% acetonitrile and 0.1% formic acid, and then the vacuum-dried peptides were dissolved in 20 µL of 0.1% (v/v) formic acid. For mass-spectrometric analysis, the peptides were loaded onto a trap column, separated using a Thermo-Dionex Ultimate 3000 HPLC system (Thermo Fisher Scientific, Waltham, MA, USA), and detected using an Orbitrap Fusion Lumos Tribrid mass spectrometer (Thermo Fisher Scientific, Waltham, MA, USA). Proteomic identification was performed by label-free quantification using Proteome Discoverer 2.3 software. The identification results were analyzed and plotted using GraphPad Prism 8.

### Example 1

This example describes the preparation and screening of nanobodies that specifically recognize and bind p62.

An alpaca-derived recombinant nanobody library (provided by the Zengpeng Li laboratory, Third Institute of Oceanography, Ministry of Natural Resources of China, and constructed according to Moutel, S. et al., NaLi-H1: A universal synthetic library of humanized nanobodies providing highly functional antibodies and intrabodies. eLife 5, 2016) was used for in vitro phage display. Human p62 protein purchased from Proteintech (catalog no. Ag13131) was used as the antigen for three rounds of panning, and the antigen concentrations used to coat the immunotubes (purchased from Wiesbaden, Germany) in the three rounds were 50 µM, 25 µM, and 25 µM, respectively.

Specifically, 1 mL of phage library (1×10¹³ pfu/mL) was added to each tube, and the tube was rotated at room temperature for 1 hour. The tubes were then washed five times with 0.1% PBST (PBS containing 0.1% Tween-20) to remove phages that had not bound to the antigen. Next, 100 mM hydrochloric acid was added, and the tubes were incubated at room temperature for 7 minutes to elute the bound phages. After neutralization with 1 M Tris buffer (pH 7.4), exponentially growing E. coli TG1 cells were infected with the eluted phages, which were reamplified, purified, and used in the next round of panning.

The phage library subjected to three rounds of panning was used to infect E. coli again, and 96 single colonies were randomly selected for phage ELISA. Clones were screened by measuring absorbance at 450 nm, and a clone having an absorbance value more than fivefold that of the milk control was identified as a positive clone. A total of 66 VHH single-domain antibodies that specifically bound human p62 were obtained, three of which were identified as capable of binding p62 protein. Their antibody sequences are set forth in SEQ ID NOs 9, 11, and 13, respectively, and their CDR sequences are shown in Table 2 below.

**Table 2: Anti-p62 single-domain antibodies**

| **Antibody No.** | **CDR1** | **CDR2** | **CDR3** |
|---|---|---|---|
| A1E | YSWEYI (SEQ ID NO 51) | RHPNDFN (SEQ ID NO 52) | VYEIISNMIKFV (SEQ ID NO 53) |
| D9A | FTYFIYV (SEQ ID NO 54) | YGNDNYF (SEQ ID NO 55) | AMIKINSKLFPY (SEQ ID NO 56) |
| E12C | FGYGWSE (SEQ ID NO 57) | SYFNVVD (SEQ ID NO 58) | VYSWNFTHI (SEQ ID NO 59) |

The affinities of the antibodies for human p62 protein were measured by BLI, and the results are shown in Fig. 1. The results showed that the affinities of the three anti-p62 single-domain antibodies (collectively referred to below as Pn) for p62 were all in the nanomolar-to-picomolar range, indicating strong binding.

### Example 2

This example describes the ability of p62 nanobodies to specifically bind, within cells, both recombinant p62 protein fused to a fluorescent tag and endogenous p62 protein.

The encoding nucleotide sequences of the three single-domain antibodies A1E, D9A, and E12C (SEQ ID NOs 10, 12, and 14) were respectively inserted between the Xho I and Apa I restriction sites of the pcDNA3.1-mCherry-4xGGS-Xho I-Apa I vector to obtain the recombinant vectors pcDNA3.1-mCherry-4xGGS-A1E, pcDNA3.1-mCherry-4xGGS-D9A, and pcDNA3.1-mCherry-4xGGS-E12C, each of which expresses a fusion protein comprising the mCherry fluorescent tag (red) and a nanobody (collectively referred to below as mCherry-Pn).

The coding nucleotide sequence of human p62 (SEQ ID NO 8) was inserted between the Xho I and Xba I restriction sites of the pcDNA3.1-EGFP-4xGGS-Xho I-Xba I vector to obtain the recombinant vector pcDNA3.1-EGFP-4xGGS-p62, which expresses the EGFP-p62 fusion protein comprising the EGFP fluorescent tag (green) and p62.

Each of the three mCherry-Pn expression plasmids was separately co-transfected with the pcDNA3.1-EGFP-4xGGS-p62 plasmid into U-2OS cells, and intracellular colocalization of mCherry-Pn with EGFP-p62 was observed. Co-transfection with pcDNA3.1-mCherry-4xGGS-Xho I-Apa I and EGFP-p62 was used as a control. After 24 hours of culture, the nuclei of live cells were stained with Hoechst 33342 (Thermo Scientific, 62249), and the cells were then observed and imaged using a Nikon laser confocal microscope. Nikon NIS image-analysis software was used to calculate the Pearson colocalization coefficient between the EGFP and mCherry signals to determine the ability of the nanobodies to bind p62 protein within cells. The results are shown in Figs. 2 and 3.

Each of the three mCherry-Pn expression plasmids was separately co-transfected with the pcDNA3.1-EGFP-4xGGS-p62 plasmid into SQSTM1-knockout HEK-293T cells (provided by the Cui Jun laboratory, Sun Yat-sen University, and generated according to Xie W. et al., OTUD7B deubiquitinates SQSTM1/p62 and promotes IRF3 degradation to regulate antiviral immunity. Autophagy, 2022). After 24 hours of culture, the nuclei of live cells were stained with Hoechst 33342 (Thermo Scientific, 62249), and the cells were then observed and imaged using a Nikon laser confocal microscope. The number of p62 droplets in each cell was measured using Nikon NIS image-analysis software. The results are shown in Fig. 4.

Figs. 2 and 3 show that all three nanobodies clearly colocalized with EGFP-p62 within cells, whereas the control mCherry fluorescent protein did not colocalize with EGFP-p62, indicating that all three nanobodies of the present invention can bind p62 protein within cells. It should be noted that Fig. 4 shows that none of the three nanobodies increased the number of intracellular EGFP droplets relative to the control expressing mCherry fluorescent protein. Because activated self-condensation of p62 protein is critical for promoting droplet formation, these results indicate that none of the three nanobodies disclosed herein can activate p62 protein upon binding thereto.

The three mCherry-Pn expression plasmids based on A1E, D9A, or E12C were also individually transfected into U-2OS cells to determine whether the nanobodies could specifically bind and recognize endogenous p62 protein. After the transfected cells were cultured for 20 hours, they were starved in Earle's Balanced Salt Solution (EBSS; Procell, PB180337), while 200 nM bafilomycin A1 (Baf A1; MCE, HY-100558) was added to block autophagy and thereby stimulate p62-body formation. After 4.5 hours, the cells were fixed with 4% paraformaldehyde and sequentially subjected to immunofluorescence staining with a rabbit anti-p62 polyclonal antibody (MBL, PM045, 1:400), an Alexa Fluor 647 goat anti-rabbit IgG fluorescent secondary antibody (Thermo Scientific, A-11035), and Hoechst 33342. The colocalization of fluorescence signals was observed in the same manner as described above, and the results are shown in Figs. 5 and 6. As shown in the figures, A1E, D9A, and E12C also recognized and specifically bound endogenous p62 protein in the cells and clearly colocalized therewith.

### Example 3

This example describes several exemplary target-binding sites on p62 recognized by p62 nanobodies and enrichment of the nanobodies in phase-separated droplets of intracellular p62 bodies.
p62 is a modular scaffold protein with multiple structural domains. Its N-terminal PB1 domain (amino acids 3-102 of SEQ ID NO 7) is involved in mediating p62 oligomerization, and its C-terminal UBA domain (amino acids 389-434 of SEQ ID NO 7) is responsible for binding ubiquitin. Both are crucial for inducing liquid-liquid phase separation and autophagic degradation of p62 (Sun, D. et al., Polyubiquitin chain-induced p62 phase separation drives autophagic cargo segregation. Cell Res 28, 405-415, 2018). In addition, amino acids 336-341 of p62 protein constitute an LC3-interacting region (LIR) motif that directs targeted binding of p62 protein to LC3 protein, which is known to be anchored to autophagic membranes (Birgisdottir, A.B. et al., The LIR motif - crucial for selective autophagy. J Cell Sci 126, 3237-3247, 2013).

A series of p62 truncation mutants were obtained by PCR using the coding nucleotide sequence of human p62 as the template to determine the target-binding sites of the nanobodies disclosed herein on p62 protein. The amplified coding sequences of the truncation variants were respectively inserted between the Xho I and Xba I restriction sites of the pcDNA3.1-EGFP-NUP98N-Xho I-Xba I vector to obtain the recombinant vectors pcDNA3.1-EGFP-NUP98N-ΔPB1, pcDNA3.1-EGFP-NUP98N-103-330, pcDNA3.1-EGFP-NUP98N-331-388, pcDNA3.1-EGFP-NUP98N-389-440, pcDNA3.1-EGFP-NUP98N-368-407, and pcDNA3.1-EGFP-NUP98N-73-132. ΔPB1 is amino acid 103-440 of SEQ ID NO 7, 103-330 is amino acid 103-330 of SEQ ID NO 7, 331-388 is amino acid 331-388 of SEQ ID NO 7, 389-440 is amino acid 389-440 of SEQ ID NO 7, 368-407 is amino acid 368-407 of SEQ ID NO 7, and 73-132 is amino acid 73-132 of SEQ ID NO 7 (see Fig. 7).

Plasmids verified by sequencing were amplified and purified, and each was separately co-transfected into U-2OS cells with one of the mCherry-Pn recombinant plasmids prepared in Example 2. The fluorescence of the coexpressed fusion proteins was observed and measured using a Nikon laser confocal microscope and NIS image-analysis software, and enrichment of the mCherry signal in the EGFP droplets was calculated as follows: $mCherry enrichment level = \left(I{\left(T\right)}_{inside droplet}/I{\left(T\right)}_{outside droplet}\right) - \left(I{\left(C\right)}_{inside droplet}/I{\left(C\right)}_{outside droplet}\right)$

Wherein, I(T) _{inside droplet} and I(T) _{outside droplet} represent the average gray value of the mCherry fluorescence signal inside ( I(T) _{inside droplet} ) or outside ( I(T) _{outside droplet} ) formed by each p62 truncated mutant, respectively; I(T) _{inside droplett} and I(T) _{outside droplet} represent the average gray value of the mCherry fluorescence signal inside ( I(T) _{inside droplet} ) or outside (I(T) _{outside droplet} ) formed by the pcDNA3.1-EGFP-NUP98N-Xho I-Xba I vector, respectively. The results are shown in Figs. 8 and 9. The three nanobodies were thus shown to bind different sites on p62 protein: A1E bound the UBA domain of p62, D9A bound amino acids 73-132 of p62, and E12C bound amino acids 368-407 of p62 (Fig. 10).

### Example 4

This example describes the preparation of an exemplary PDF-Bin based on a p62 nanobody and the use of the PDF-Bin to recruit an exemplary target protein (POI) to p62 bodies for targeted degradation.

First, a cell line that can stably express EGFP-POI was constructed. TDP43 is a widely expressed DNA/RNA-binding protein that is closely associated with amyotrophic lateral sclerosis (ALS) and frontotemporal dementia (FTD) (Keating, SS et al., TDP-43 pathology: From noxious assembly to therapeutic removal. Prog Neurobiol 211, 102229, 2022). Pathological aggregates of TDP43 in the cytoplasm are closely associated with damage to neurons and brain regions, and also drive the disruption of neuromuscular junctions in disease model mice and ALS patients (Neumann, M. et al., Ubiquitinated TDP-43 in frontotemporal lobar degeneration and amyotrophic lateral sclerosis. Science 314, 130-133, 2006; Altman, T. et al., Axonal TDP-43 condensates drive neuromuscular junction disruption through inhibition of local synthesis of nuclear encoded mitochondrial proteins. Nat Commun 12, 6914, 2021). In addition, TDP43 has a typical intrinsically disordered region at its N terminus and can oligomerize under physiological conditions, thereby promoting its own liquid-liquid phase separation (Carter GC et al., N-terminal Domain of TDP43 Enhances Liquid-Liquid Phase Separation of Globular Proteins. J Mol Biol. 2021 May 14;433(10):166948.).

The recombinant lentiviral vector pLVX-EGFP-TP43 containing the EGFP-TDP43 coding nucleotide sequence (SEQ ID NO 18) was co-transfected with the packaging plasmids pMD2.G (Addgene, 12259) and psPAX2 (Addgene, 12260) into HEK-293T cells to obtain an infectious lentiviral supernatant. HEK-293T cells and U-2OS cells were infected with lentiviral culture medium, and the following two stable cell lines were obtained through resistance selection and flow cytometry sorting: HEK-293T/EGFP-TDP43 cells and U-2OS/EGFP-TDP43 cells.

A GFP nanobody capable of specifically binding GFP fluorescent protein (referred to below as Gn) has been reported, and its amino acid sequence and encoding nucleotide sequence are set forth in SEQ ID NOs 19 and 20, respectively; see, for example, Kubala, M.H. et al., Structural and thermodynamic analysis of the GFP:GFP-nanobody complex. Protein Sci 19, 2389-2401, 2010. Using the nanobody Gn as the second binding specificity, the following bispecific fusion proteins of the present invention were constructed by conventional molecular cloning techniques: A1E fused to Gn (A1E-Gn), D9A fused to Gn (D9A-Gn), and E12C fused to Gn (E12C-Gn). Their amino acid sequences are set forth in SEQ ID NOs 21 to 23, respectively, and their encoding nucleotide sequences are set forth in SEQ ID NOs 24 to 26, respectively. The nucleotide sequence encoding Gn and the nucleotide sequences encoding the three Pn-Gn proteins described above were respectively inserted between the Xho I and Apa I restriction sites of the pcDNA3.1-mCherry-4xGGS-Xho I-Apa I vector to obtain the recombinant vectors pcDNA3.1-mCherry-4xGGS-Gn, pcDNA3.1-mCherry-4xGGS-A1E-Gn, pcDNA3.1-mCherry-4xGGS-D9A-Gn, and pcDNA3.1-mCherry-4xGGS-E12C-Gn, which respectively express the fusion proteins mCherry-Gn, mCherry-A1E-Gn, mCherry-D9A-Gn, and mCherry-E12C-Gn. The resulting bispecific fusion proteins have both Gn-based binding activity that specifically targets an EGFP-fused target protein (EGFP-POI) and Pn-based binding activity that specifically targets p62 protein, and are collectively referred to herein as PDF-Bin.

The recombinant vectors pcDNA3.1-mCherry-4xGGS-Gn, pcDNA3.1-mCherry-4xGGS-A1E-Gn, pcDNA3.1-mCherry-4xGGS-D9A-Gn, and pcDNA3.1-mCherry-4xGGS-E12C-Gn, the blank vector pcDNA3.1-mCherry-4xGGS-Xho I-Apa I, and the recombinant vectors encoding the Pn nanobodies prepared in Example 2, namely pcDNA3.1-mCherry-4xGGS-A1E, pcDNA3.1-mCherry-4xGGS-D9A, and pcDNA3.1-mCherry-4xGGS-E12C, were each separately transfected into The cells were then subjected to immunofluorescence staining with a rabbit anti-p62 polyclonal antibody (MBL, PM045, 1:400), an Alexa Fluor 647 goat anti-rabbit IgG fluorescent secondary antibody (Thermo Scientific, A-11035), and Hoechst 33342. The cells were observed and imaged using a Nikon laser confocal microscope, and colocalization of the EGFP, mCherry, and Alexa Fluor 647 fluorescence signals was analyzed using Nikon NIS image-analysis software. The results are shown in Figs. 11 to 14. The fluorescence-colocalization results showed that no colocalization among a nanobody, EGFP-TDP43, and p62 was observed when a vector expressing Pn or Gn was transfected alone (Figs. 11 and 12), whereas clear colocalization was observed when a vector expressing a PDF-Bin based on A1E or E12C was transfected alone, indicating that these PDF-Bins could effectively recruit EGFP-POI to p62 bodies (Figs. 13 and 14). No obvious ternary complex formation was observed when D9A-based PDF-Bin was transfected alone. However, after further co-transfection of the recombinant vector pcDNA3.1-EGFP-4xGGS-p62 into the cells to overexpress the p62 protein, the ternary complex formed by the nanobody, EGFP-TDP43 and p62 was detected. The results are shown in Fig. 15.

Without wishing to be bound by theory, the above results are considered to indicate that, although the fusion protein disclosed herein does not comprise a ligand capable of directly activating p62 protein as one of its binding specificities, it can recruit TDP43 and p62 protein to form a spatially proximal ternary complex, exploit and further amplify the liquid-liquid phase-separation property of TDP43 protein having an intrinsically disordered region, and ultimately promote oligomerization and activation of the bound p62 protein to form observable and detectable p62 bodies.

The same vectors described above (a blank vector, a recombinant vector expressing only one nanobody, and a recombinant vector expressing the bispecific binding molecule PDF-Bin of the present invention) were each separately transfected into the HEK-293T/EGFP-TDP43 stable cell line. After 48 hours of culture, total cellular protein was extracted, and intracellular EGFP-TDP43 levels were measured by Western blotting. For Western blotting, the membrane was first incubated with a rabbit anti-p62 polyclonal antibody and an HRP-conjugated goat anti-rabbit secondary antibody. After the results were visualized and imaged, the membrane was re-probed with a rabbit anti-GAPDH polyclonal antibody and an HRP-conjugated goat anti-rabbit secondary antibody and was visualized and imaged again. The results are shown in Fig. 16 (left). The grayscale intensities of the Western blot bands were quantified using ImageJ FIJI. After normalization to the grayscale intensities of the GAPDH bands, the data were analyzed using GraphPad Prism 8, and the results are shown in Fig. 16 (right).

The Western blot results showed that the extent of EGFP-TDP43 degradation was significantly increased in cells transfected with a PDF-Bin based on A1E, D9A, or E12C. In view of the fluorescence-colocalization results described above, it is considered that, unlike AUTOTAC molecules, which require a p62-activating ligand as one of the two targeting warheads, the PDF-Bin disclosed herein can promote p62-body formation merely by forming a ternary complex and thereby promote degradation of the target protein via the p62 bodies.

### Example 5

This example describes the use of an exemplary PDF-Bin to control targeted degradation of multiple different types of target proteins.

Using conventional molecular cloning methods, the EGFP-TDP43 coding sequence in the pLVX-EGFP-TDP43 vector of Example 2 was replaced with a coding sequence of the target protein G3BP1, hnRNPK, or HTT-Q103 bearing N-terminal EGFP, or with a coding sequence of the target protein TSPAN4, PD-L1, or APP bearing C-terminal EGFP, thereby constructing the series of recombinant vectors shown in Table 3 below.

**Table 3:**

| **Recombinant vector** | **Target protein** | **Target Protein Type** | **Target protein coding sequence** | **Intrinsically disordered protein or protein having an intrinsically disordered region?** |
|---|---|---|---|---|
| pLVX-EGFP | EGFP | Soluble cytoplasmic protein & soluble nuclear protein | SEQ ID NO 3 | No |
| pLVX-EGFP-G3BP1 | G3BP1 | Cytoplasmic protein | SEQ ID NO 29 | Yes |
| pLVX-EGFP-hnRNPK | hnRNPK | Nuclear protein | SEQ ID NO 31 | Yes |
| pLVX-EGFP-HTT-Q103 | HTT-Q103 | Cytoplasmic protein that forms insoluble aggregates | SEQ ID NO 33 | Yes |
| pLVX-TSPAN4-EGFP | TSPAN4 | Membrane protein | SEQ ID NO 35 | No |
| pLVX-PD-L1-EGFP | PD-L1 | Membrane protein | SEQ ID NO 37 | No |
| pLVX-APP-EGFP | APP | Membrane protein | SEQ ID NO 27 | No |
| pLVX-EGFP-TDP43 | TDP43 | Soluble nuclear protein/cytoplasmic aggregate | SEQ ID NO 17 | Yes |

The coding fragment of mCherry-4xGGS-A1E-Gn from Example 4 was amplified by PCR and homologously recombined between the EcoRI and AgeI restriction sites of the pLVX-TETONE-EcoRI-AgeI vector to obtain the pLVX-TETONE-mCherry-4xGGS-A1E-Gn recombinant vector. This vector can generate lentivirus with the help of packaging plasmids and express the mCherry-A1E-Gn fusion protein under DOX induction.

Using the pLVX-TETONE-mCherry-4xGGS-A1E-Gn recombinant vector, an infectious lentiviral supernatant was prepared by the same method as in Example 4 and was used to separately transduce HEK-293T cells and U-2OS cells. The following two stable cell lines were ultimately obtained by antibiotic selection and flow cytometric sorting: HEK-293T/A1E-Gn^{TRE} cells and U-2OS/A1E-Gn^{TRE} cells, both of which express the mCherry-A1E-Gn fusion protein upon DOX induction.

The recombinant vectors pLVX-EGFP, pLVX-EGFP-G3BP1, pLVX-EGFP-hnRNPK, pLVX-EGFP-HTT-Q103, pLVX-TSPAN4-EGFP, pLVX-PD-L1-EGFP, and pLVX-APP-EGFP were transfected into U-2OS/A1E-Gn^{TRE} cells, respectively. After 6 hours, the medium was replaced with fresh medium containing 1 µg/mL DOX or the same volume of DMSO, and the cells were cultured for an additional 24 hours. The cells were then subjected to immunofluorescence staining using a rabbit anti-p62 polyclonal antibody and an Alexa Fluor 647 goat anti-rabbit IgG fluorescent secondary antibody. Observation and imaging were performed using a Nikon laser confocal microscope, and the Pearson colocalization coefficients of EGFP and Alexa Fluor 647 fluorescence signals were analyzed using Nikon NIS image analysis software. The results are shown in Figs. 17A-17H and Fig. 18.

Laser confocal imaging showed that, when individually transfected, all target proteins exhibited separate distributions and did not colocalize with p62 bodies. After mCherry-A1E-Gn (PDF-Bin) expression was induced by DOX, all EGFP-POIs except EGFP were enriched in p62 bodies and formed colocalized ternary complexes with PDF-Bin. Without wishing to be bound by theory, this may be because EGFP protein is highly ordered and readily soluble and has little or no tendency to undergo liquid-liquid phase separation under physiological conditions. Therefore, PDF-Bin was not observed to promote the formation of visible p62 bodies in normal cells having a low level of autophagy. In contrast, intrinsically disordered proteins, proteins having intrinsically disordered regions, and membrane proteins all tend to undergo phase separation or membrane anchoring under physiological conditions. Thus, for a variety of different target proteins, the PDF-Bin disclosed herein can exploit these properties of the target proteins to effectively promote p62-body formation and produce distinct colocalized puncta.

The eight recombinant vectors described above, namely pLVX-EGFP, pLVX-EGFP-G3BP1, pLVX-EGFP-hnRNPK, pLVX-EGFP-HTT-Q103, pLVX-TSPAN4-EGFP, pLVX-PD-L1-EGFP, and pLVX-APP-EGFP, were also separately transfected into HEK-293T/A1E-GnTRE cells or, as a control, wild-type HEK-293T cells. Six hours later, the culture medium was replaced with fresh medium containing 1 µg/mL DOX or the same volume of DMSO, and the culture was continued for another 48 hours. Total cellular protein was then extracted, and Western blotting was performed by the same method as in Example 4 using a rabbit anti-EGFP polyclonal antibody or a rabbit anti-mCherry polyclonal antibody as the primary antibody for the first incubation. The results are shown in Fig. 19A. For the electrophoretic bands in the figure, ImageJ FIJI was used for quantitative analysis of gray values to calculate the degree of EGFP-POI degradation as shown below: $Degree of degradation = \left(1-\frac{Grayscale intensity of the band after DOX treatment}{Grayscale intensity of the band without DOX treatment}\right) ∗ 100 %$

All grayscale intensities were normalized to the grayscale intensity of the GAPDH band and analyzed using GraphPad Prism 8. The results are shown in Fig. 19B.

Consistent with the fluorescence-colocalization results, the Western blot results described above show that, while promoting formation of a target protein-fusion protein-p62 ternary complex, the PDF-Bin disclosed herein also significantly increases, to varying extents, the efficiency of targeted degradation of target proteins having intrinsically disordered regions. Without wishing to be bound by theory, the effect of targeted degradation mediated by the multispecific fusion protein PDF-Bin disclosed herein is considered likely to be associated with the degree of disorder of the target protein and its tendency to undergo liquid-liquid phase separation/self-condensation. A protein having a high degree of intrinsic disorder readily triggers liquid-liquid phase separation and self-condensation and therefore may be subject to pronounced targeted degradation when a multispecific fusion protein disclosed herein is used. Such a protein is therefore a favorable druggable target for the multispecific fusion proteins disclosed herein, whereas this effect is difficult to achieve using an activating ligand that requires an ordered structure as a binding site or a small-molecule compound as a targeting warhead.

### Example 6

This example describes a kinetic study of targeted degradation of a target protein mediated by an exemplary PDF-Bin.

The pLVX-EGFP-TDP43 plasmid was introduced into the U-2OS/A1E-Gn^{TRE} cells prepared in Example 5, and the stable monoclonal cell line U-2OS/EGFP-TDP43;A1E-Gn^{TRE} was ultimately obtained.

The stable cell line was seeded in a 12-well culture plate and cultured for 12-18 hours, after which the medium was replaced with fresh medium containing 1 µg/mL DOX or a corresponding volume of DMSO, and the cells were cultured for an additional 24 hours. A portion of the cells was then used to extract total cellular protein for Western blotting by the same method as in Example 4 (using a rabbit anti-EGFP, rabbit anti-p62, or rabbit anti-mCherry polyclonal antibody as the primary antibody and an HRP-conjugated goat anti-rabbit secondary antibody); another portion of the cells was used to extract total cellular RNA using an RNA Rapid Extraction Kit (MF159) for qPCR. Two independent replicates were performed per gene and three independent replicates per treatment. RPL13A was used as the normalization reference, and the normalized fold change for each target gene was calculated. The results are shown in Fig. 20. The results showed that the cell line could stably and continuously express the EGFP-TDP43 fusion protein, and the expression level of the mCherry-A1E-Gn fusion protein could be significantly increased under DOX induction (data not shown). Furthermore, no statistically significant effect of A1E-Gn on the expression of EGFP-TDP43 and other proteins was observed at the transcriptional (mRNA) level.

The stable cell line was seeded in a 12-well culture plate and cultured for 12-18 hours, after which the medium was replaced with fresh medium containing 1 µg/mL DOX or a corresponding volume of DMSO, and the cells were cultured for an additional 24 hours. The Pearson colocalization coefficient between the EGFP and Alexa Fluor 647 fluorescence signals was determined using the same confocal fluorescence microscopy and analysis methods as in Example 3, and the number of p62 bodies was also counted. The results are shown in Figs. 21A and 21B. In addition, the stable cells in which the medium had been replaced as described above were cultured for an additional 48 hours and then analyzed by immunoprecipitation coupled with mass spectrometry (IP-MS). The results are shown in Fig. 22.

Confocal fluorescence imaging and IP-MS showed that induction of A1E-Gn expression by DOX promoted formation of a ternary complex comprising EGFP-TDP43, mCherry-A1E-Gn, and p62, indicating that A1E-Gn could recruit EGFP-TDP43 to p62 bodies (Fig. 21A). Meanwhile, the number of p62 bodies increased significantly by approximately sixfold (Fig. 21B), indicating that increasing the interaction between TDP43 and p62 could promote liquid-liquid phase separation of p62 and thereby facilitate the subsequent degradation process. Moreover, after DOX was added, A1E-Gn was observed to recruit only TDP43 to p62 bodies (Fig. 22), with no recruitment of proteins other than TDP43, indicating that the PDF-Bin disclosed herein has high specificity as a targeted protein degradation tool.

The kinetics of A1E-Gn-mediated targeted degradation of EGFP-TDP43 were then characterized in a series of time-course and DOX dose-response experiments (Figs. 23 to 28). The stable cells described above were seeded in 12-well culture plates and cultured for 12-18 hours, after which the medium was replaced with fresh medium containing 1 µg/mL DOX, and the cells were cultured for an additional 12, 24, 36, 48, 60, or 72 hours. Alternatively, the medium was replaced with fresh medium containing a specified concentration of DOX (0, 0.025, 0.05, 0.1, 0.25, 0.5, 0.75, 1, 2.5, 5, 7.5, or 10 µg/mL), and the cells were cultured for an additional 48 hours. Changes in the cellular fluorescence signals were observed by laser confocal microscopy, and the results are shown in Figs. 23 and 24. Total cellular protein was extracted and analyzed by Western blotting, and the results are shown in Figs. 25 and 26. The results showed that the EGFP-TDP43 fluorescence signal was significantly reduced as early as 12 hours after DOX was added (Figs. 23 and 24), and the target protein reached the maximum extent of degradation after 36-48 hours (Figs. 25 and 26). Furthermore, the results of the concentration gradient experiments showed that the A1E-Gn-mediated degradation of the target protein was also concentration-dependent, and the degradation effect increased with increasing DOX concentration (Figs. 27 and 28).

### Example 7

This example describes a study of the pathway of target-protein degradation mediated by an exemplary PDF-Bin.

The stable monoclonal cell line U-2OS/EGFP-TDP43;A1E-Gn^{TRE} prepared in Example 6 was seeded in a 12-well culture plate and cultured for 12-18 hours. The medium was then replaced with fresh medium containing 1 µg/mL DOX or a corresponding volume of DMSO, and the cells were cultured for an additional 12 hours. Then, 1 µM proteasome inhibitor MG-132 or 200 nM lysosomal acidification inhibitor bafilomycin A1 (Baf A1) was added to the specified experimental group, and the cells were cultured for an additional 36 hours. Total protein was then extracted from the cells and subjected to Western blot analysis. The results are shown in Fig. 29.

The results showed that the EGFP-POI level did not change significantly upon Baf A1 treatment, whereas the EGFP-POI level increased significantly after MG-132 treatment, indicating that a proteasome inhibitor could significantly inhibit A1E-Gn-mediated targeted degradation. When a proteasome inhibitor and an autophagy inhibitor were added simultaneously, target-protein degradation could be almost completely inhibited (Fig. 30). These results indicate that A1E-Gn-mediated degradation primarily depends on the proteasomal pathway.

Furthermore, Figs. 31 to 33 show that the protein level of p62 was significantly increased under DOX treatment, but its mRNA content did not change in a statistically significant manner. This indicates that A1E-Gn can also reduce the degradation of p62 protein itself, leading to the accumulation of more p62 protein in cells. This phenomenon is consistent with A1E-Gn-mediated degradation being primarily dependent on the proteasomal pathway. This may be because interaction with PDF-Bin causes p62 protein to participate more extensively in the proteasomal degradation pathway, thereby sparing it from degradation in autolysosomes.

### Example 8

This example describes the preparation of another exemplary PDF-Bin molecule and characterization of its properties associated with targeted degradation of a target protein.

Signal transducer and activator of transcription 3 (STAT3) is a convergence node for many cancer-related signaling pathways. It is widely expressed in various cell types and is activated by interleukins, interferons, growth factors, and kinases such as SRC and MET. STAT3 is constitutively activated in a variety of cancers, including non-small cell lung cancer (NSCLC), melanoma, lymphoma, and leukemia, as well as in non-cancerous cells in the tumor microenvironment, and plays a key role in regulating cancer cell proliferation, differentiation, and angiogenesis. Although STAT3 is a promising target for cancer therapy, it is generally recognized as having poor druggability. To date, only a few antisense oligonucleotides (ASOs) or PROTACs targeting STAT3 have been developed that can effectively reduce the mRNA or protein levels of STAT3 and have shown some antitumor activity. The C-terminal transactivation domain (TAD) of STAT3 has been reported to be highly disordered, with more than 50 amino acid residues forming an intrinsically disordered region (see Jacopo Sgrignani et a*l.*, *S*tructural Biology of STAT3 and Its Implications for Anticancer Therapies Development*. Int. J. Mol. Sci*. 2018, 19(6), 1591). In this example, endogenous STAT3 protein in tumor cells was selected as the target to prepare another exemplary PDF-Bin, in order to investigate the degradation efficacy of the PDF-Bin molecules disclosed herein against an endogenous target and their antitumor potential.

The previously reported STAT3 antibody mimetic (monobody) MS3-6 was used in this example (La Sala, G. et al., Selective inhibition of STAT3 signaling using monobodies targeting the coiled-coil and N-terminal domains. Nat Commun 11, 4115, 2020); MS3-6 specifically recognizes and binds the core fragment of STAT3. A nucleotide fragment encoding a fusion protein comprising the EGFP fluorescent tag, the p62 nanobody A1E, and MS3-6 (SEQ ID NO 39), and a nucleotide fragment encoding a fusion protein comprising EGFP, A1E, and the mCherry-specific nanobody Cn (SEQ ID NO 16) were respectively inserted between the EcoR I and Age I restriction sites of the pLVX-TETONE-EcoR I-Age I vector to obtain the recombinant vectors pLVX-TETONE-EGFP-A1E-MS3-6 and pLVX-TETONE-EGFP-A1E-Cn. With the aid of packaging plasmids, these vectors can produce lentivirus and, upon DOX induction, respectively express the EGFP-A1E-MS3-6 and EGFP-A1E-Cn fusion proteins.

Each recombinant vector described above was co-transfected with the packaging plasmids into HEK-293T cells to obtain an infectious lentiviral supernatant. A549 cells were transduced with the lentiviral supernatants, and the A549/EGFP-A1E-MS3-6^{TRE} and A549/EGFP-A1E-Cn^{TRE} cell lines were ultimately obtained by antibiotic selection and flow cytometric sorting.

### Immunofluorescence Localization by Confocal Microscopy

A549/EGFP-A1E-MS3-6^{TRE} cells were seeded in a four-well glass-bottom culture dish and cultured for 12-18 hours, after which the medium was replaced with fresh medium containing 1 µg/mL DOX or a corresponding volume of DMSO, and the cells were cultured for an additional 24 hours. The cells were subjected to immunofluorescence staining using a rabbit anti-STAT3 polyclonal antibody and an Alexa Fluor 546 goat anti-rabbit IgG fluorescent secondary antibody, as well as a mouse anti-p62 monoclonal antibody and an Alexa Fluor 647 goat anti-mouse IgG fluorescent secondary antibody. The cells were observed and imaged using a Nikon laser confocal microscope. Nikon NIS image-analysis software was used to analyze enrichment of the STAT3 fluorescence signal in p62 bodies and the number of p62 bodies, and the data were analyzed using GraphPad Prism 8. The results are shown in Figs. 34 to 36.

Enrichment of the STAT3 fluorescence signal was calculated as follows: $Enrichment level of STAT 3 fluorescence signal = \frac{I \left(in\right)}{I \left(out\right)}$

*I(in)* represents the mean fluorescence intensity of STAT3 inside the p62 bodies, and *I(out)* represents mean fluorescence intensity of STAT3 outside the p62 bodies (intracellular).

The results showed that, after A1E-MS3-6 expression was induced by DOX, STAT3 was significantly recruited to p62 bodies (Figs. 34 to 36), and the number of p62 bodies increased significantly (Fig. 36), demonstrating that A1E-MS3-6 could interact with p62 and STAT3 protein to form a ternary complex and that the liquid-liquid phase-separation property of STAT3 promoted formation of p62 condensates.

### Time- and Concentration-Dependent Degradation

The A549/EGFP-A1E-MS3-6^{TRE} stable cells were then seeded in 12-well culture plates and cultured for 12-18 hours. The medium was replaced with fresh medium containing 1 µg/mL DOX, and the cells were cultured for an additional 6, 12, 18, 24, 30, 36, 42, or 48 hours. Alternatively, the medium was replaced with fresh medium containing a specified concentration of DOX (0, 0.05, 0.1, 0.25, 0.5, or 1 µg/mL), and the cells were cultured for an additional 48 hours. Total cellular protein was then extracted and analyzed by Western blotting. The results are shown in Figs. 37 to 40. The results showed that the level of endogenous STAT3 protein began to decrease 6 hours after the addition of DOX (Figs. 37 and 38). Target-protein degradation mediated by this exemplary PDF-Bin was also DOX-dose-dependent (Figs. 39 and 40).

### Identification of the Degradation Pathway Using Degradation Inhibitors

The pathway of A1E-MS3-6-mediated targeted degradation of STAT3 was also investigated by the same method as in Example 7. A549/EGFP-A1E-MS3-6^{TRE} stable cells were seeded in a 12-well culture plate and cultured for 12-18 hours, after which the medium was replaced with fresh medium containing 1 µg/mL DOX or a corresponding volume of DMSO, and the cells were cultured for an additional 12 hours. Then, 1 µM proteasome inhibitor MG-132 or 200 nM lysosomal acidification inhibitor bafilomycin A1 (Baf A1) was added to the specified experimental group, and the cells were cultured for an additional 36 hours. Total cellular protein was then extracted and analyzed by Western blotting. The results are shown in Figs. 41 and 42. As shown in the figure, unlike the case of EGFP-TDP43 degradation, proteasome inhibitor treatment had no statistically significant effect on endogenous STAT3 content. On the contrary, Baf A1, as an autophagy inhibitor, could significantly inhibit STAT3 degradation. This indicates that A1E-MS3-6-mediated degradation mainly depends on the autophagy-lysosome pathway. This difference is considered likely to result from the localization of inactive STAT3 in the cytoplasm, where p62 primarily functions as a selective autophagy receptor to mediate degradation.

### Half-Life of p62 Protein

A549/EGFP-A1E-MS3-6^{TRE} stable cells were seeded in a 12-well culture plate and cultured for 12-18 hours, after which the medium was replaced with fresh medium containing 1 µg/mL DOX or a corresponding volume of DMSO, and the cells were cultured for an additional 12 hours. The cells were then treated with 100 µg/mL cycloheximide (CHX), a protein-synthesis inhibitor, for 4, 8, or 12 hours; 0 hours corresponds to no CHX treatment. Total cellular protein was then extracted and analyzed by Western blotting. The Western blot results were visualized by the same method as in Example 4, and the grayscale intensity of the p62 band was quantified. All grayscale intensities were normalized to that of the GAPDH band. The results are shown in Figs. 43 and 44.

CHX treatment excluded the effect of p62 protein synthesis on the Western blot results, showing that DOX-induced expression of PDF-Bin significantly shortened the intracellular half-life of p62 protein and indicating that A1E-MS3-6 could significantly increase clearance of p62 itself. This phenomenon is consistent with A1E-MS3-6-mediated degradation being primarily dependent on the lysosomal pathway, because more p62 protein participates in STAT3 degradation through the lysosomal pathway, thereby also shortening the half-life of p62 itself.

### Example 9

This example describes the inhibitory effect on tumor cells following treatment with an exemplary PDF-Bin of the present invention.

Specifically, the non-small cell lung cancer cell line A549 was treated with EGFP-A1E-MS3-6 as described above, and the fusion protein of A1E and anti-mCherry nanobody (A1E-Cn) was used as a control (Fridy, PC et al., A robust pipeline for rapid production of versatile nanobody repertoires. Nat Methods 11, 1253-1260, 2014). The migration rate of the tumor cells was evaluated by a scratch assay, and their proliferative capacity was evaluated by a colony formation assay.

### Scratch Assay

A549/EGFP-A1E-MS3-6^{TRE} stable cells were seeded into 6-well plates and cultured for 12-18 hours. The culture medium was then replaced with fresh medium containing the specified concentration of DOX or the corresponding volume of DMSO. The cells were cultured for 48 hours until they reached 90-100% confluence. A scratch was then made in each well using a 200-µL pipette tip. The medium was replaced with DMEM containing 2% fetal bovine serum, 50 µg/mL penicillin/streptomycin, and the corresponding concentration of DOX or DMSO. Images of the scratch were immediately acquired using a Nikon inverted microscope with a 4× objective, and this time point was designated 0 hours. After imaging, the cells were cultured further and imaged again after 24 hours, which was designated 24 hours. The results are shown in Fig. 45. The scratch area was measured using ImageJ FIJI, and the wound healing rate was calculated using the following formula, with the wound healing efficiency then normalized: $Wound healing efficiency \left(%\right) = \frac{{S}_{t = 0 h} - {S}_{t = 24 h}}{{S}_{t = 0 h}} \times 100$ Where S ₍ₜ₌₀ₕ₎ and S ₍ₜ₌₂₄ₕ₎ represent the wound area at 0 hours and 24 hours, respectively. Graphs were generated and statistical significance was calculated using GraphPad Prism 8. The results are shown in Fig. 47 (left).

### Colony Formation Assay

A549/EGFP-A1E-MS3-6^{TRE} stable cells were seeded at 400 cells/well into 12-well plates and cultured for 12-18 hours. The culture medium was then replaced with fresh medium containing the specified concentration of DOX or the corresponding volume of DMSO. The DOX-containing medium was replaced every 48 hours to prevent loss of DOX activity. After 9 days of DOX treatment, the cells were fixed with 4% paraformaldehyde at room temperature for 15 minutes and then stained with crystal violet staining solution for 10-20 minutes, until cell colonies were visible to the naked eye and appeared dark purple. The cells were rinsed three times with ultrapure water and then imaged using a SONY IMX800 camera. The results are shown in Fig. 46. Colonies were counted using ImageJ, and graphs were generated and statistical significance was calculated using GraphPad Prism 8. The results are shown in Fig. 47 (right).

As shown by the results above, both the migratory and proliferative capacities of tumor cells treated with A1 E-MS3-6 were significantly reduced relative to tumor cells treated with the control molecule (Fig. 47), indicating that the PDF-Bin disclosed herein has potential as an antitumor agent or even a therapeutic agent.

### Example 10

This example describes how the induced-degradation capability of a PDF-Bin molecule of the present application can be enhanced by an oligomerization element or a phase-separation element.

The following PDF-Bin molecules were prepared using the same method as in Example 4:
PmSE10 (SEQ ID NO 67), a monobody that specifically targets human p62 protein, and Tm12D (SEQ ID NO 77), a monobody that specifically targets human TDP43 protein, were fused through the flexible linker set forth in SEQ ID NO 50 to form a PDF-Bin molecule referred to below as PmSE10-Tm12D;
PnA5 (SEQ ID NO 72), a nanobody that specifically targets human p62 protein, and Tm12D were fused through the flexible linker set forth in SEQ ID NO 50 to form a PDF-Bin molecule referred to below as PnA5-Tm12D.

Each of the following oligomerization elements or phase-separation elements was fused to the C terminus of PmSE10-Tm12D or PnA5-Tm12D through a flexible linker to obtain enhanced PDF-Bin molecules:
tetrameric oligomerization domain, SEQ ID NO 60;
14-mer oligomerization domain, SEQ ID NO 61;
60-mer oligomerization domain, SEQ ID NO 62;
NUP98_IDR, SEQ ID NO 63;
FUS_IDR, SEQ ID NO 64;
SFPQ_IDR, SEQ ID NO 65;
P2S2, SEQ ID NO 66.

The nucleotide sequences encoding the PDF-Bin molecules and enhanced PDF-Bin molecules described above were respectively inserted between the Xho I and Apa I restriction sites of the pcDNA3.1-mCherry-4xGGS-Xho I-Apa I vector to express the respective PDF-Bin molecules. The recombinant vectors described above and the blank vector pcDNA3.1-mCherry-4xGGS-Xho I-Apa I were then separately transfected into U-2OS cells, and the cells were fixed after 48 hours. The cells were then subjected to immunofluorescence staining with a rabbit anti-TDP43 polyclonal antibody (Proteintech, 10782-2-AP, 1:400), a mouse anti-p62 polyclonal antibody (MBL, M162-3, 1:400), an Alexa Fluor 488 goat anti-rabbit IgG fluorescent secondary antibody (A-11034, 1:200), an Alexa Fluor 647 goat anti-mouse IgG fluorescent secondary antibody (Thermo Scientific, A-21236, 1:200), and Hoechst 33342. The cells were observed and imaged using a Nikon laser confocal microscope. The mean fluorescence intensities of the Alexa Fluor 488 and mCherry signals were analyzed using Nikon NIS image-analysis software, and graphs were generated and nonlinear fitting was performed using GraphPad Prism 8. The results are shown in Figs. 48 to 50.

As shown in the figures, tandem linkage of a PDF-Bin molecule of the present application to an oligomerization element or a phase-separation element further enhanced condensation and activation of p62 bodies, and the magnitude of the enhancement exhibited a degree of dependence on the degree of oligomerization or the strength of phase separation. These enhanced PDF-Bin molecules can increase targeted degradation activity against target proteins and, as should be understood, will also enhance regulation of target-protein-related pathways, for example, tumor-cell-killing activity.

The present invention has been described in detail above. For those skilled in the art, the present invention can be practiced in a wide range of ways with equivalent parameters, concentrations and conditions without departing from the spirit and scope of the invention and without the need for unnecessary experiments. Although specific examples of the present invention have been provided, it should be understood that further modifications may be made to the present invention. In summary, in accordance with the principles of the present invention, the present application is intended to encompass any variation, use, or modification of the present invention, including departures from the scope disclosed herein that involve conventional techniques known in the art. Some of the basic features can be applied within the scope of the appended claims.

## Claims

1. A multispecific fusion protein comprising at least one first affinity peptide that specifically binds to an autophagy receptor p62 protein and at least one second affinity peptide that specifically binds to a target, wherein the first affinity peptide and the second affinity peptide are optionally covalently linked through a linking sequence/linker.

2. The fusion protein of claim 1, which, in the presence of p62 protein and the target, is capable of forming a ternary complex comprising the fusion protein, p62 protein, and the target;
optionally, the fusion protein increases liquid-liquid phase separation of p62 protein relative to a condition in which the fusion protein is absent,
optionally, the increase in liquid-liquid phase separation is selected from any one or any combination of the following: an increase in the number of liquid-liquid phase-separation droplets containing p62 protein, an increase in the area of liquid-liquid phase-separation droplets containing p62 protein, an increase in the degree of oligomerization of p62 protein in liquid-liquid phase-separation droplets containing p62 protein, and a decrease in the fluidity of p62 protein in liquid-liquid phase-separation droplets containing p62 protein;
optionally, the target is an intrinsically disordered protein, a protein having an intrinsically disordered region, or a protein aggregate, membrane complex, and/or organelle containing, as a monomer or component, an intrinsically disordered protein or a protein having an intrinsically disordered region;
optionally, the intrinsically disordered region of the protein having an intrinsically disordered region comprises about 30 or more consecutive amino acid residues;
optionally, the target is selected from a cytoplasmic protein, a nuclear protein, a membrane protein, or an organelle;
optionally, the target is selected from the group consisting of TDP43, hnRNPK, G3BP1, HTT-Q103, TSPAN4, PD-L1, APP, and STAT3;
optionally, the first affinity peptide and the second affinity peptide are each independently selected from a nanobody or a synthetic binding protein;
optionally, the nanobody is selected from a single-domain antibody, a single-chain antibody (scFv), a minibody, a half-antibody, or an antigen-binding fragment of an antibody; preferably, the single-domain antibody is selected from a V_{H}H domain antibody, a heavy-chain variable domain (VH) antibody, a V_{NAR} domain antibody, and a V_{L} domain antibody;
optionally, the synthetic binding protein is selected from a monobody, an affibody, an anticalin, or a DARPin;
optionally, the first affinity peptide comprises CDR1, CDR2, and CDR3 contained in any one of SEQ ID NO 9, 11, 13, 67, or 72;
optionally, CDR1, CDR2, and CDR3 comprised in the first affinity peptide respectively have: the amino acid sequences set forth in SEQ ID NOs 51 to 53; the amino acid sequences set forth in SEQ ID NOs 54 to 56; the amino acid sequences set forth in SEQ ID NOs 57 to 59; the amino acid sequences set forth in SEQ ID NOs 68 to 70; or the amino acid sequences set forth in SEQ ID NOs 73 to 75;
optionally, the first affinity peptide comprises the amino acid sequence set forth in any one of SEQ ID NOs 9, 11, 13, 67, and 72;
optionally, the second affinity peptide comprises CDR1, CDR2 and CDR3 contained in SEQ ID NO 19 or 77;
optionally, CDR1, CDR2, and CDR3 comprised in the second affinity peptide respectively have: the amino acid sequences set forth in SEQ ID NOs 78 to 80, respectively; or the amino acid sequences set forth in SEQ ID NOs 81 to 83, respectively;
optionally, the second affinity peptide comprises the amino acid sequence set forth in SEQ ID NO 19 or 77;
optionally, the linking sequence or linker is a rigid or flexible linker, preferably a flexible linker, and more preferably a linker comprising the sequence set forth in SEQ ID NO 50;
optionally, the fusion protein further comprises an oligomerization element, wherein the oligomerization element is directly covalently linked to the first affinity peptide and/or the second affinity peptide, or is covalently linked thereto through a linking sequence/linker;
optionally, each fusion protein comprises one or more oligomerization elements, preferably one oligomerization element;
optionally, the oligomerization element is a dimeric or higher-order oligomerization domain, preferably a tetrameric or higher-order oligomerization domain, more preferably an octameric or higher-order oligomerization domain, and most preferably a 12-polymer or higher-order oligomerization domain;
optionally, the oligomerization element comprises an amino acid sequence set forth in any one of SEQ ID NOs 60 to 62, an amino acid sequence having at least 90% identity to an amino acid sequence set forth in any one of SEQ ID NOs 60 to 62, or an amino acid sequence having one or several deletions, substitutions, or additions relative to an amino acid sequence set forth in any one of SEQ ID NOs 60 to 62;
optionally, the fusion protein further comprises a phase-separation element, wherein the phase-separation element is directly covalently linked to the first affinity peptide and/or the second affinity peptide, or is covalently linked thereto through a linking sequence/linker;
optionally, each fusion protein comprises one or more phase-separation elements;
optionally, the phase-separation element comprises an amino acid sequence set forth in any one of SEQ ID NOs 63 to 66, an amino acid sequence having at least 90% identity to an amino acid sequence set forth in any one of SEQ ID NOs 63 to 66, or an amino acid sequence having one or several deletions, substitutions, or additions relative to an amino acid sequence set forth in any one of SEQ ID NOs 63 to 66;
optionally, the fusion protein further comprises an element that facilitates delivery, preferably an element that facilitates delivery to a target site, wherein the element is optionally selected from a cell-penetrating peptide, a nuclear localization signal, an organelle localization signal, an endoplasmic reticulum retention signal, a peroxisomal targeting signal, and a mitochondrial targeting peptide;
optionally, the fusion protein comprises one first affinity peptide and one second affinity peptide, or one first affinity peptide and two or more identical or different second affinity peptides, or two or more identical or different first affinity peptides and one second affinity peptide, or two or more identical or different first affinity peptides and two or more identical or different second affinity peptides.

3. A nucleic acid comprising a nucleotide sequence encoding the fusion protein of claim 1 or 2.
optionally, the encoding nucleotide sequence is operably linked to a promoter.

4. A recombinant vector comprising the nucleic acid of claim 3, wherein the vector is optionally a recombinant viral vector.

5. An engineered cell that expresses the fusion protein of claim 1 or 2 or comprises the nucleic acid of claim 3 or the recombinant vector of claim 4.

6. A method for producing a fusion protein, wherein the method comprises
expressing the nucleic acid of claim 3 or the recombinant vector of claim 4 in a suitable host cell, or
culturing the engineered cell of claim 5, and purifying the expressed fusion protein.

7. A composition, wherein the composition comprises the fusion protein of claim 1 or 2, the nucleic acid of claim 3, the recombinant vector of claim 4, or the engineered cell of claim 5.

8. A method for degrading a target in a cell, wherein the method comprises
expressing the fusion protein of claim 1 or 2, the nucleic acid of claim 3, or the recombinant vector of claim 4 in the cell, such that the amount of the target is reduced relative to a condition in which the fusion protein, the nucleic acid, or the recombinant vector is absent, preferably by at least about 30% to about 90%.

9. A method for preventing and/or treating a disease, wherein the method comprises
administering to a subject the fusion protein of claim 1 or 2, the nucleic acid of claim 3, the recombinant vector of claim 4, the engineered cell of claim 5, or the composition of claim 7,
optionally, the disease is selected from diseases associated with protein misfolding, misaggregation, or aberrant production,
preferably, the disease is selected from neurodegenerative diseases, cardiovascular diseases, neuromuscular diseases, tumors, metabolic diseases, or autoimmune diseases,
optionally, the neurodegenerative disease is selected from Alzheimer's disease, Huntington's disease, Parkinson's disease, amyotrophic lateral sclerosis, hereditary ataxia, Vici syndrome, or BPAN syndrome,
optionally, the cardiovascular disease is selected from coronary artery disease, atherosclerosis, or pulmonary hypertension,
optionally, the neuromuscular disease is selected from hereditary cardiomyopathy, distal myopathy, muscular dystrophy, congenital myopathy, spinal muscular atrophies (SMAs), motor neuron disease, Duchenne muscular dystrophy, Becker muscular dystrophy, limb-girdle muscular dystrophy, myotonic dystrophy, myotubular myopathy, centronuclear myopathy, nemaline myopathy, selenoprotein N-related myopathy, Pompe disease, glycogen storage disease III, or amyotrophic lateral sclerosis,
optionally, the tumor is selected from lung cancer, colorectal cancer, head and neck cancer (e.g., head and neck squamous cell carcinoma), glioma (e.g., neuroglioma), neuroblastoma, melanoma, breast cancer, bladder cancer, kidney cancer, ovarian cancer, pancreatic cancer, cervical cancer, esophageal cancer, sarcoma, esophageal cancer (e.g., esophageal squamous cell carcinoma), thyroid cancer (e.g., papillary thyroid carcinoma), prostate cancer, B-cell lymphoma, chronic lymphocytic leukemia (CLL), chronic myeloid leukemia, diffuse large B-cell lymphoma (DLBCL), follicular lymphoma, myelodysplastic syndrome (MDS), non-Hodgkin lymphoma (NHL), acute lymphoblastic leukemia (ALL), acute monocytic leukemia, multiple myeloma, acute myeloid leukemia (AML), mixed-lineage leukemia, NUT midline carcinoma, Burkitt lymphoma, mycosis fungoides (MF), or a metastatic form thereof,
optionally, the metabolic disease is selected from hyperlipidemia, atherosclerosis, non-alcoholic fatty liver disease, or diabetes,
optionally, the autoimmune disease is selected from systemic lupus erythematosus, atopic dermatitis, myasthenia gravis, type I diabetes, sarcoidosis, asthma, graft-versus-host disease, autoimmune arthritis, rheumatoid arthritis, Sjogren's syndrome, psoriasis, multiple sclerosis, inflammatory bowel disease, ulcerative colitis, Crohn's disease, uveitis, or polychondritis.

10. A method for screening multispecific fusion proteins, comprising the steps of:
(a) obtaining one or more first affinity peptides capable of specifically binding to an autophagy receptor p62 protein and one or more second affinity peptides capable of specifically binding to a target, and optionally covalently linking at least one of the first affinity peptides to at least one of the second affinity peptides through a linking sequence or linker to obtain a candidate fusion protein library;
(b) selecting, from the library, a candidate fusion protein that significantly increases colocalization of p62 protein with the target relative to a control in which the candidate fusion protein is absent;
(c) selecting, from the candidate fusion proteins selected in step (b), a fusion protein that increases liquid-liquid phase separation of p62 protein relative to a control in which the candidate fusion protein is absent;
optionally, the increase in liquid-liquid phase separation in step (c) is selected from any one or any combination of the following: an increase in the number of liquid-liquid phase-separation droplets containing p62 protein, an increase in the area of liquid-liquid phase-separation droplets containing p62 protein, an increase in the degree of oligomerization of p62 protein in liquid-liquid phase-separation droplets containing p62 protein, and a decrease in the fluidity of p62 protein in liquid-liquid phase-separation droplets containing p62 protein;
optionally, the target is an intrinsically disordered protein, a protein having an intrinsically disordered region, a protein containing an intrinsically disordered protein or a protein having an intrinsically disordered region, or a protein aggregate, membrane complex, and/or organelle containing, as a monomer or component, an intrinsically disordered protein or a protein having an intrinsically disordered region;
optionally, the target is selected from a cytoplasmic protein, a nuclear protein, a membrane protein, or an organelle;
optionally, the target is selected from the group consisting of TDP43, hnRNPK, G3BP1, HTT-Q103, TSPAN4, PD-L1, APP, and STAT3;
optionally, the method further comprises step (d): determining the level of the target and selecting a fusion protein for which the amount of the target is reduced relative to a control in which the candidate fusion protein is absent; preferably, the amount of the target is reduced by at least about 30% to about 90%;
optionally, the first affinity peptide and the second affinity peptide are each independently selected from a nanobody or a synthetic binding protein;
optionally, the nanobody is selected from a single-domain antibody, a single-chain antibody (scFv), a minibody, a half-antibody, or an antigen-binding fragment of an antibody; preferably, the single-domain antibody is selected from a V_{H}H domain antibody, a heavy-chain variable domain (VH) antibody, a V_{NAR} domain antibody, and a V_{L} domain antibody;
optionally, the synthetic binding protein is selected from monobody, affibody, anticalin, or DARPin;
optionally, the first affinity peptide comprises CDR1, CDR2, and CDR3 contained in any one of SEQ ID NO 9, 11, 13, 67, or 72;
optionally, CDR1, CDR2, and CDR3 comprised in the first affinity peptide respectively have: the amino acid sequences set forth in SEQ ID NOs 51 to 53; the amino acid sequences set forth in SEQ ID NOs 54 to 56; the amino acid sequences set forth in SEQ ID NOs 57 to 59; the amino acid sequences set forth in SEQ ID NOs 68 to 70; or the amino acid sequences set forth in SEQ ID NOs 73 to 75;
optionally, the first affinity peptide comprises the amino acid sequence set forth in any one of SEQ ID NOs 9, 11, 13, 67, and 72;
optionally, the second affinity peptide comprises CDR1, CDR2, and CDR3 contained in SEQ ID NO 19 or 77;
optionally, CDR1, CDR2, and CDR3 comprised in the second affinity peptide respectively have the amino acid sequences set forth in SEQ ID NOs 78 to 80 or the amino acid sequences set forth in SEQ ID NOs 81 to 83;
optionally, the first affinity peptide comprises the amino acid sequence set forth in SEQ ID NO 19 or 77;
optionally, the linker connecting the first affinity peptide and the second affinity peptide in step (b) comprises the sequence set forth in SEQ ID NO 50.

11. The fusion protein obtained by the method of claim 10.

12. A nanobody comprising CDR1, CDR2, and CDR3 contained in any one of SEQ ID NOs 9, 11, 13, 67, 72, 19, and 77,
preferably, CDR1, CDR2, and CDR3 comprised therein respectively have:
the amino acid sequences set forth in SEQ ID NOs 51 to 53;
the amino acid sequences set forth in SEQ ID NOs 54 to 56;
the amino acid sequences set forth in SEQ ID NOs 57 to 59;
the amino acid sequences set forth in SEQ ID NOs 68 to 70;
the amino acid sequences set forth in SEQ ID NOs 73 to 75;
the amino acid sequences set forth in SEQ ID NOs 78 to 80; or
the amino acid sequences set forth in SEQ ID NOs 81 to 83.

13. A nucleic acid encoding the affinity peptide of claim 12, wherein the encoding nucleotide sequence preferably comprises the nucleotide sequence set forth in any one of SEQ ID NOs 10, 12, 14, 71, and 76.
